# EUROPEAN PATENT SPECIFICATION

(11) **EP 2 600 838 B1**
(45) Date of publication and mention of the grant of the patent: **16.09.2015**
(21) Application number: 11746176.4
(22) Date of filing: 04.08.2011
(51) Int. Cl.: A61K 9/107, A61K 9/48, A61K 47/10, A61K 47/12, A61K 47/14, A61K 47/26, A61K 31/407

(54) **PHARMACEUTICAL DOSAGE FORM COMPRISING 6'-FLUORO-(N-METHYL- OR N,N-DIMETHYL-)-4-PHENYL-4',9'-DIHYDRO-3'H-SPIRO[CYCLOHEXANE-1,1'-PYRANO[3,4,B]INDOL]-4-AMINE**
DARREICHUNGSFORM MIT 6'-FLUOR-(N-METHYL- ODER N,N-DIMETHYL)-4-PHENYL-4',9'-DIHYDRO-3'H-SPIRO[CYCLOHEXAN-1,1'-PYRANO[3,4,B]INDOL]-4-AMIN
FORME GALÉNIQUE PHARMACEUTIQUE COMPRENANT 6'-FLUORO-(N-MÉTHYL- OR N,N-DIMÉTHYL-)-4-PHÉNYL-4',9'-DIHYDRO-3'H-SPIRO[CYCLOHEXANE-1,1'-PYRANO[3,4,B]INDOL]-4-AMINE

(30) Priority: 04.08.2010 US 370634 P; 04.08.2010 EP 10008115
(43) Date of publication of application: 12.06.2013
(73) Proprietor: Grünenthal GmbH, 52078 Aachen (DE)
(72) Inventor: GRÜNING, Nadja, 52064 Aachen (DE); SCHILLER, Marc, 52076 Aachen (DE)
(86) International application number: PCT/EP2011/003918
(87) International publication number: WO 2012/016703

(56) References cited:
- WO-A1-2004/043967
- WO-A1-2006/082099
- WO-A1-2008/040481

## Description

### FIELD OF THE INVENTION

*The invention relates to a pharmaceutical dosage form for use in the treatment of pain, which contains a pharmacologically active agent according to general formula (I)* 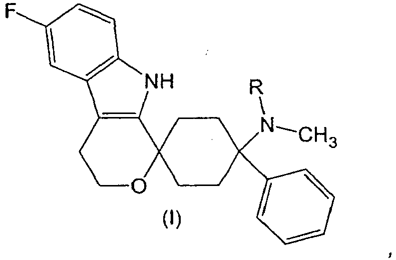 *wherein R is -H or -CH₃,*
*or a physiologically acceptable salt thereof, and* a *self-emulsifying formulation;*
*wherein the pharmaceutical dosage form is administered twice daily, once daily or less frequently*."

The pharmacologically active agents according to general formula (I) can also be referred to as 6'-Fluoro-(N-methyl- or N,N-dimethyl-)-4-phenyl-4',9'-dihydro-3'H-spiro[cyclohexane-1,1'-pyrano[3,4,b]indol]-4-amine. Unless expressly stated otherwise, this term also includes the physiologically acceptable salts.

### BACKGROUND OF THE INVENTION

The pharmacologically active agents according to the invention are known from the prior art and can be administered orally, perorally, parenterally, intravenously, intraperitoneally, intradermally, intramuscularly, intranasally, buccally, rectally or locally, for example to the skin, the mucous membranes or into the eyes. The compounds exhibit analgesic properties and are particularly suitable for the treatment of acute, visceral, neuropathic or chronic pain (cf., e.g., WO 2004/043967 and WO 2008/040481).

WO 2006/082099 *relates to a form of administration with a minimum breaking strength of 400 N while releasing the physiologically effective substance at least partly in a delayed manner in physiological conditions*.

Conventional analgesics are typically available as formulations providing immediate release or as formulations providing prolonged release.

On the one hand, formulations providing immediate release upon oral administration have the advantage that they lead to a fast release of the analgesic in the gastrointestinal tract. As a result, a comparatively high dose of the analgesic is quickly absorbed leading to high plasma levels within a short period of time and resulting in a rapid onset of pain relief, i.e. analgesic action begins shortly after administration. This is particularly desirable in acute pain.

At the same time, however, a rapid reduction in the analgesic action is usually observed, because metabolization and/or excretion of the analgesic cause a decrease of its plasma levels. For that reason, formulations providing immediate release of analgesics typically need to be administered frequently, e.g. eight times per day. This is not only detrimental with respect to patient compliance but also may cause comparatively high peak plasma drug concentrations and high fluctuations between peak and trough plasma drug concentrations which in turn may deteriorate tolerability.

On the other hand, formulations providing prolonged release upon oral administration have the advantage that they need to be administered less frequently, typically once daily or twice daily. This improves patient compliance and also can reduce peak plasma drug concentrations and fluctuations between peak and trough plasma drug concentrations which in turn may improve tolerability.

At the same time, however, release of the analgesic in the gastrointestinal tract is prolonged. As a result, a comparatively low dose of the analgesic is quickly absorbed leading to low plasma levels and resulting in a retarded onset of pain relief, i.e. analgesic action begins quite a while after first administration.

Furthermore, as formulations providing prolonged release typically contain higher doses of the analgesics than formulations providing immediate release, they bear a higher risk of being misused. Older patients in particular frequently have difficulties in taking solid pharmaceutical dosage forms. To counter this problem, various apparatuses have been developed by means of which solid pharmaceutical dosage forms may be comminuted or pulverized ("tablet crushers"). Such apparatuses are used, for example, by the care staff in old people's homes. The pharmaceutical dosage forms are then administered to the people being cared for not as tablets etc. but rather as powder, for example to get round the difficulties involved in swallowing tablets. However, the comminution of pharmaceutical dosage forms with such apparatuses is problematic if the pharmaceutical dosage forms are prolonged release formulations. As a rule, comminution then results in destruction of the inner structure of the pharmaceutical dosage form, which is responsible for the prolonged release, so doing away with the prolonged-release action. Consequently, after administration, frequently all the physiologically active substance originally contained in the pharmaceutical dosage form is released in a relatively short time, whereby a comparatively very high plasma concentration of the substance is abruptly reached for a relatively short period (dose dumping). In this way, the original prolonged-release formulations become immediate-release formulations. Depending on the physiological activity of the substance, this may cause considerable side-effects however, and in extreme cases may even lead to the death of the patient (cf., e.g., J. E. Mitchell, Oral Pharmaceutical dosage forms That Should Not Be Crushed: 2000 Update, Hospital Pharmacy, 2000; H. Miller et al., To Crush or Not to Crush, Nursing 2000; R. Griffith et al., Tablet Crushing and the law: the implications for nursing; Prof. Nurse 2003). Intentional chewing of prolonged-release formulations may also lead to an overdose of the substance contained therein. Sometimes patients chew the pharmaceutical dosage forms deliberately, though often in ignorance of the type and purpose of a prolonged-release formulation, because they hope for a quicker effect.

Formulations providing a dual release mode, i.e. a combination of immediate release with prolonged release, are also known (cf., e.g., C.M. Lopez et al., Compressed Matrix Core Tablet as a Quick/Slow Dual-Component Delivery System Containing Ibuprofen, AAPS PharmSciTech 2007; 8(3), E1-E8). However, these formulations typically rely upon immediate-release units and prolonged-release units that are locally separated from one another and therefore, such pharmaceutical dosage forms can only be prepared by specific and costly methods.

It is an object of the invention to provide pharmaceutical dosage forms containing 6'-Fluoro-(N-methyl- or N,N-dimethyl-)-4-phenyl-4',9'-dihydro-3'H-spiro[cyclohexane-1,1'-pyrano[3,4,b]-indol]-4-amine which have advantages compared to the pharmaceutical dosage forms of the prior art. In particular, the pharmaceutical dosage forms should provide good bioavailability and rapid pain relief already after the first administration, but also should have a high tolerability, good compliance, and safety.

This object has been achieved by the subject-matter of the patent claims.

It has been surprisingly found that 6'-Fluoro-(N-methyl- or N,N-dimethyl-)-4-phenyl-4',9'-dihydro-3'H-spiro[cyclohexane-1,1'-pyrano[3,4,b]indol]-4-amine has a comparatively poor water solubility. Further, it has been surprisingly found that in spite of said poor water solubility, pharmaceutical dosage forms can be prepared which provide immediate release of 6'-Fluoro-(N-methyl- or N,N-dimethyl-)-4-phenyl-4',9'-dihydro-3'H-spiro[cyclohexane-1,1'-pyrano[3,4,b]indol]-4-amine and provide good bioavailability. Still further, it has been surprisingly found that 6'-Fluoro-(N-methyl- or N,N-dimethyl-)-4-phenyl-4',9'-dihydro-3'H-spiro[cyclohexane-1,1'-pyrano[3,4,b]indol]-4-amine has a relatively large pharmacokinetic half life time (t_{1/2}) and thus, provides pharmacological activity for a comparatively extended period of time after administration.

Therefore, it has been surprisingly found that upon preferably oral administration of the pharmaceutical dosage form containing the pharmacologically active agent according to the invention, a rapid onset of pain relief can be achieved followed by a prolonged analgesic effect, although, or even if, the pharmaceutical dosage form provides immediate release. Therefore, the pharmaceutical dosage form according to the invention combines the advantageous properties of conventional formulations providing immediate release - rapid pain relief due to adequately high concentration of active ingredient just after administration of the pharmaceutical composition - with the advantageous properties of conventional formulations providing prolonged release - long-lasting analgesic action owing to an adequately high level of active ingredient over a prolonged time -, and at the same time even overcomes the drawbacks of said conventional formulations. By taking the pharmacologically active agent in the formulation according to the invention, the patient can effectively combat his pain acutely and, at the same time, treat if effectively over a prolonged period without further measures and merely by regular administration at 12 (or e.g., 24) hourly intervals.

It is particularly surprising that the pharmaceutical dosage form according to the invention not only allows the pharmacologically active agent to start flowing rapidly in the plasma when the pharmaceutical dosage form is first administered, leading to a rapid onset of pain relief in the patient owing to the immediate release, but at the same time ensures long-lasting therapeutic efficacy over a relatively long period (at least 12 hours). Therefore, the pain suffered by a patient can rapidly be alleviated when the pharmaceutical dosage form according to the invention is administered without the analgesic action quickly fading again.

The pharmaceutical dosage form according to the invention has good patient compliance and safety. Even if the pharmaceutical dosage form according to the invention is tampered with, e.g. by means of tablet crushers, dose dumping cannot occur - crushing the pharmaceutical dosage form does not further accelerate the immediate release profile.

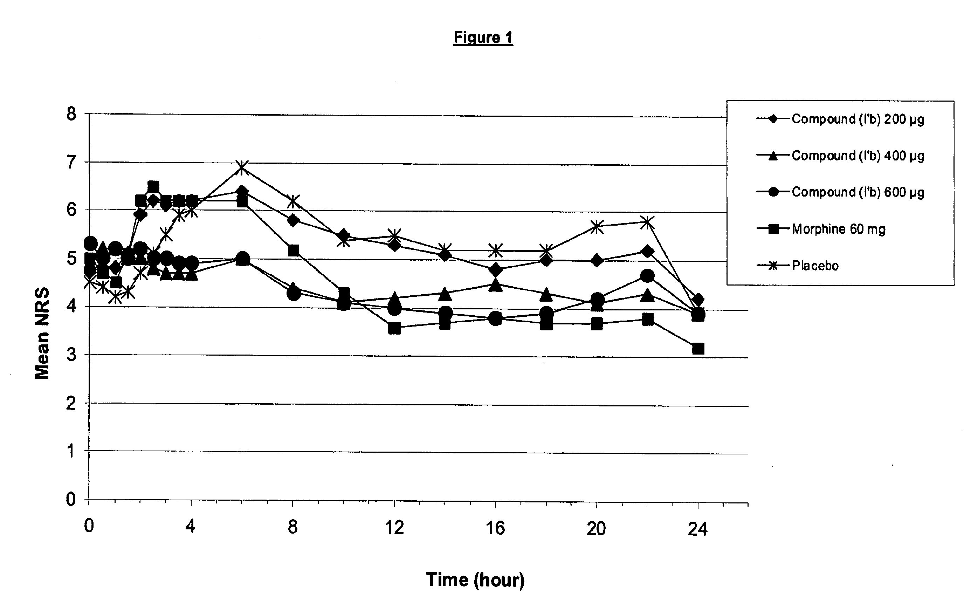

Figure 1 shows the averaged numerical rating scale (NRS) values measured over a 24 hour period after administration of different single doses of the compound according to formula (I'b) (200, 400, 600 µg) compared to morphine and placebo in patients with acute postoperative pain following orthopedic surgery (bunionectomy).

*The invention relates to a pharmaceutical dosage form for use in the treatment of pain, which contains a pharmacologically active agent according to general formula (I)* 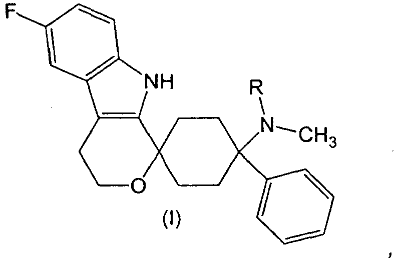 *wherein R is -H or-CH₃,*
*or a physiologically acceptable salt thereof, and* a *self-emulsifying formulation;*
*wherein the pharmaceutical dosage form is administered twice daily, once daily or less frequently.*

The pharmacologically active agent according to general formula (I) can also be referred to as "6'-fluoro-N-methyl-4-phenyl-4',9'-dihydro-3'H-spiro[cyclohexane-1,1'-pyrano[3,4,b]indol]-4-amine" when R is -H, and "6'-fluoro-N,N-dimethyl-4-phenyl-4',9'-dihydro-3'H-spiro[cyclohexane-1,1'-pyrano[3,4,b]indol]-4-amine" when R is -CH₃; for the purpose of the specification, the pharmacologically active agent according to general formula (I) can also be referred to as "6'-fluoro-(N-methyl- or N,N-dimethyl-)-4-phenyl-4',9'-dihydro-3'H-spiro-[cyclohexane-1,1'-pyrano[3,4,b]indol]-4-amine".

In a preferred embodiment, the pharmacologically active agent according to general formula (I) has a stereochemistry according to general formula (I') 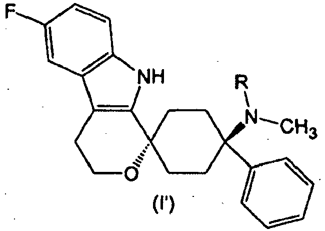 wherein R is -H or-CH₃, or a physiologically acceptable salt thereof.

In another embodiment of the pharmaceutical dosage form according to the invention, the compound of formula (I) is selected from 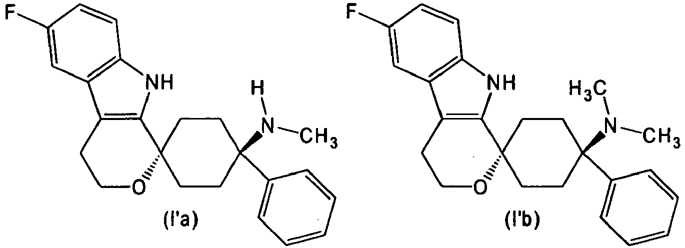 in the form of the free base or a physiologically acceptable salt thereof.

The free base according to general formula (I'a) can be systematically referred to as "1,1-(3-methylamino-3-phenylpentamethylene)-6-fluoro-1,3,4,9-tetrahydropyrano[3,4-b]indole (trans)" or as "(1r,4r)-6'-fluoro-N-methyl-4-phenyl-4',9'-dihydro-3'H-spiro[cyclohexane-1,1'-pyrano[3,4,b]indol]-4-amine", respectively.

The free base according to general formula (I'b) can be systematically referred to as "1,1-(3-dimethylamino-3-phenylpentamethylene)-6-fluoro-1,3,4,9-tetrahydropyrano[3,4-b]indole (trans)" or as "(1r,4r)-6'-fluoro-N,N-dimethyl-4-phenyl-4',9'-dihydro-3'H-spiro[cyclohexane-1,1'-pyrano[3,4,b]indol]-4-amine", respectively.

The definition of the pharmacologically active agent according to general formula (I) as used herein includes 6'-fluoro-(N-methyl- or N,N-dimethyl-)-4-phenyl-4',9'-dihydro-3'H-spiro-[cyclohexane-1,1'-pyrano[3,4,b]indol]-4-amine, derivatives thereof and stereoisomers thereof in any possible form, thereby particularly including solvates and polymorphs, salts, in particular acid addition salts and corresponding solvates and polymorphs.

In a preferred embodiment, the pharmacologically active agent according to general formula (I) is present as the single diastereomer according to general formula (I').

In another preferred embodiment the pharmacologically active agent according to general formula (I) is present as mixture of diastereomers. Such a mixture may contain the diastereomers in any ratio. A diastereomeric mixture could, for example, contain the diastereomers in a ratio of 60±5:40±5, 70±5:30±5, 80±5:20±5 or 90±5:10±5. Preferably, the pharmaceutical dosage form according to the invention contains the diastereomer according to general formula (I') in a diastereomeric excess (de) of at least 50%de, more preferably at least 60%de, still more preferably at least 70%de, yet more preferably at least 80%de, even more preferably at least 90%de, most preferably at least 95%de, and in particular at least 98%de, with respect to the other diastereomer (i.e. trans vs. cis and anti vs. syn, respectively).

6'-Fluoro-(N-methyl- or N,N-dimethyl-)-4-phenyl-4',9'-dihydro-3'H-spiro[cyclohexane-1,1'-pyrano[3,4,b]indol]-4-amine may be present in the pharmaceutical dosage form according to the invention in form of the free base or in form of an acid addition salt, whereby any suitable acid capable of forming such an addition salt may be used.

The conversion of 6'-Fluoro-(N-methyl- or N,N-dimethyl-)-4-phenyl-4',9'-dihydro-3'H-spiro-[cyclohexane-1,1'-pyrano[3,4,b]indol]-4-amine into a corresponding addition salt, for example, via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example, diethyl ether, diisopropyl ether, alkyl acetates, acetone and/or 2-butanone. Moreover, trimethylchlorosilane in aqueous solution is also suitable for the preparation of hydrochlorides.

The pharmacologically active agent according to general formula (I) is contained in the pharmaceutical dosage form in a therapeutically effective amount. The amount that constitutes a therapeutically effective amount varies according to the compound, the condition being treated, the severity of said condition, the patient being treated, and whether the pharmaceutical dosage form is designed for an immediate or retarded release.

In a preferred embodiment, the content of the pharmacologically active agent according to the general formula (I) in the pharmaceutical dosage form according to the invention is at most 95 wt.-%, more preferably at most 50 wt.-%, yet more preferably at most 25 wt.-%, still more preferably at most 10 wt.-%, even more preferably at most 5 wt.-%, most preferably at most 1.0 wt.-%, and in particular at most 0.5 wt.-%.

In another preferred embodiment, the content of the pharmacologically active agent according to the general formula (I) in the pharmaceutical dosage form according to the invention is at least 0.001 wt.-%, more preferably at least 0.005 wt.-%, yet more preferably at least 0.01 wt.-%, still more preferably at least 0.05 wt.-%, even more preferably at least 0.1 wt.-%, most preferably at least 0.5 wt.-%, and in particular at least 1.0 wt.-%.

Unless explicitly stated otherwise, in the meaning of the present invention the indication "wt.-%" shall mean weight of the respective ingredient per total weight of the pharmaceutical dosage form. In case that the pharmaceutical dosage form is film coated or encapsulated by an encapsulating medium which does not contain any amount of the pharmacologically active agent according to the general formula (I) and surrounds a core that in turn contains the total amount of the pharmacologically active agent according to the general formula (I), the indication "wt.-%" shall mean weight of the respective ingredient per total weight of the composition forming said core.

When the pharmaceutical dosage form is encapsulated or film coated, the pharmacologically active agent according to general formula (I) is preferably homogeneously distributed in the core of the pharmaceutical dosage form. Preferably, the encapsulating medium or film coating does not contain any pharmacologically active agent according to general formula (I).

The dose of the pharmacologically active agent according to general formula (I) preferably is in the range of 0.1 µg to 5000 µg, more preferably in the range of 0.1 µg to 1000 µg, and most preferably in the range of 1.0 µg to 100 µg or in the range of 30 µg to 600 µg.

In a preferred embodiment, the content of the pharmacologically active agent according to general formula (I) in the pharmaceutical dosage form is within the range of 25±20 µg, more preferably 25±15 µg, still more preferably 25±10 µg, and most preferably 25±5 µg.

In another preferred embodiment, the content of the pharmacologically active agent according to general formula (I) in the pharmaceutical dosage form is within the range of 40±35 µg, more preferably 40±30 µg, still more preferably 40±25 µg, yet more preferably 40±20 µg, even more preferably 40±15 µg, most preferably 40±10 µg, and in particular 40±5 µg.

In still another preferred embodiment, the content of the pharmacologically active agent according to general formula (I) in the pharmaceutical dosage form is within the range of 50±35 µg, more preferably 50±30 µg, still more preferably 50±25 µg, yet more preferably 50±20 µg, even more preferably 50±15 µg, most preferably 50±10 µg, and in particular 50±5 µg.

In yet another preferred embodiment, the content of the pharmacologically active agent according to general formula (I) in the pharmaceutical dosage form is within the range of 60±35 µg, more preferably 60±30 µg, still more preferably 60±25 µg, yet more preferably 60±20 µg, even more preferably 60±15 µg, most preferably 60±10 µg, and in particular 60±5 µg.

In another preferred embodiment, the content of the pharmacologically active agent according to general formula (I) in the pharmaceutical dosage form is within the range of 100±90 µg, more preferably 100±80 µg, still more preferably 100±60 µg, yet more preferably 100±40 µg, even more preferably 100±20 µg, most preferably 100±10 µg, and in particular 100±5 µg.

In still another preferred embodiment, the content of the pharmacologically active agent according to general formula (I) in the pharmaceutical dosage form is within the range of 200±175 µg, more preferably 200±150 µg, still more preferably 200±125 µg, yet more preferably 200±100 µg, even more preferably 200±75 µg, most preferably 200±50 µg, and in particular 200±25 µg.

In yet another preferred embodiment, the content of the pharmacologically active agent according to general formula (I) in the pharmaceutical dosage form is within the range of 400±350 µg, more preferably 400±300 µg, still more preferably 400±250 µg, yet more preferably 400±200 µg, even more preferably 400±150 µg, most preferably 400±100 µg, and in particular 400±60 µg.

In a preferred embodiment the pharmaceutical dosage form is for use in the treatment of acute pain, where the dose of the pharmacologically active agent according to general formula (I) preferably is in the range of 50 µg to 3000 µg, more preferably in the range of 100 µg to 1000 µg, even more preferably in the range of 300 µg to 500 µg, and most preferably in the range of 350 µg to 450 µg.

In another preferred embodiment, the pharmaceutical dosage form is for use in the treatment of acute pain, where the dose of the pharmacologically active agent according to general formula (I) preferably is in the range of 200 µg to 400 µg, and in particular in the range of 250 µg to 350 µg.

In a preferred embodiment, the pharmaceutical dosage form is for use in the treatment of acute pain, where the dose of the pharmacologically active agent according to general formula (I) preferably is in the range of 200 µg to 400 µg, and in particular in the range of 250 µg to 350 µg.

In a preferred embodiment, the pharmaceutical dosage form is for use in the treatment of acute pain, where the dose of the pharmacologically active agent according to general formula (I) preferably is in the range of 250 µg to 450 µg, and in particular in the range of 300 µg to 400 µg.

In another preferred embodiment, the pharmaceutical dosage form is for use in the treatment of acute pain, where the dose of the pharmacologically active agent according to general formula (I) preferably is in the range of 300 µg to 500 µg, and in particular in the range of 350 µg to 450 µg.

In yet another preferred embodiment, the pharmaceutical dosage form is for use in the treatment of acute pain, where the dose of the pharmacologically active agent according to general formula (I) preferably is in the range of 350 µg to 550 µg, and in particular in the range of 400 µg to 500 µg.

In even another preferred embodiment, the pharmaceutical dosage form is for use in the treatment of acute pain, where the dose of the pharmacologically active agent according to general formula (I) preferably is in the range of 400 µg to 600 µg, and in particular in the range of 450 µg to 550 µg.

In another preferred embodiment the pharmaceutical dosage form is for use in the treatment of chronic pain, where the dose of the pharmacologically active agent according to general formula (I) preferably is in the range of 0.1 µg to 500 µg, more preferably in the range of 1 µg to 250 µg, even more preferably in the range of 5 µg to 100 µg, and most preferably in the range of 10 µg to 50 µg.

In a preferred embodiment, the pharmaceutical dosage form according to the invention is for oral administration, i.e. the pharmaceutical dosage form is adapted for oral administration. Suitable alternative pathways of administration of the pharmaceutical dosage form according to the invention include but are not limited to vaginal and rectal administration.

The pharmaceutical dosage form according to the invention is for administration twice daily, once daily or less frequently, i.e. the pharmaceutical dosage form is adapted for administration twice daily, once daily or less frequently.

In a preferred embodiment, the pharmaceutical dosage form according to the invention is for administration twice daily.

For the purpose of the specification, "administration twice daily" (bid) preferably means that the pharmaceutical dosage form is adapted for being administered according to a regimen comprising the administration of a first pharmaceutical dosage form according to the invention and the subsequent administration of a second pharmaceutical dosage form according to the invention, wherein both, the first and the second pharmaceutical dosage form are administered during a time interval of about 24 hours, but wherein the second pharmaceutical dosage form is administered not earlier than 6 hours, preferably not earlier than 8 hours, more preferably not earlier than 10 hours and in particular, about 12 hours after the first pharmaceutical dosage form has been administered.

In another preferred embodiment, the pharmaceutical dosage form according to the invention is for administration once daily.

For the purpose of the specification, "administration once daily" (sid) preferably means that the pharmaceutical dosage form is adapted for being administered according to a regimen comprising the administration of a first pharmaceutical dosage form according to the invention and the subsequent administration of a second pharmaceutical dosage form according to the invention, wherein both, the first and the second pharmaceutical dosage form are administered during a time interval of about 48 hours, but wherein the second pharmaceutical dosage form is administered not earlier than 18 hours, preferably not earlier than 20 hours, more preferably not earlier than 22 hours and in particular, about 24 hours after the first pharmaceutical dosage form has been administered.

In another preferred embodiment, the pharmaceutical dosage form according to the invention is for administration once daily or less frequently.

In another preferred embodiment, the pharmaceutical dosage form according to the invention is for administration less frequently than once daily, preferably thrice during four days (3/4), twice during three days (2/3), thrice during five days (3/5), once during two days (1/2), thrice in a week (3/7), twice during five days (2/5), once during three days (1/3), twice in a week (2/7), once during four days (1/4), once during five days (1/5), once during six days (1/6), or once in a week (1/7). According to this embodiment, administration once during two days (1/2) is particularly preferred.

A skilled person is fully aware that administration regimens "twice daily, once daily, or less frequently" may be realized by administering a single pharmaceutical dosage form containing the full amount of the pharmacologically active agent according to general formula (I) to be administered at a particular point in time or, alternatively, administering a multitude of dose units, i.e. two, three or more dose units, the sum of which multitude of dose units containing the full amount of the pharmacologically active agent according to general formula (I) to be administered at said particular point in time, where the individual dose units are for simultaneous administration or administration within a short period of time, e.g. within 5, 10 or 15 minutes.

Preferably, the pharmaceutical dosage form according to the invention provides immediate release of the pharmacologically active agent according to general formula (I). Preferably, the pharmaceutical dosage form is specifically designed to provide immediate release of the pharmacologically active agent according to general formula (I) *in vitro* in accordance with Ph. Eur. When the pharmaceutical dosage form is coated, e.g., with a coating that is soluble in gastric juice, the release kinetic is preferably monitored after such coating has been dissolved.

For the purpose of specification, the term "immediate release" refers to any release profile that fulfills at least one, preferably both, of the following requirements. First, the pharmaceutical dosage form disintegrates in 10 minutes or less following exposure to a disintegrating medium. Methods to determine the disintegration time are known to a person skilled in the art. For instance, they can be determined according to the USP XXIV disintegration test procedure, using, for example, an Erweka ZT-71 disintegration tester. Second, the pharmaceutical dosage form releases at least 70 wt.-% of the drug within 15 minutes following exposure to a dissolution medium. Preferably, the *in vitro* release properties of the pharmaceutical dosage form according to the invention are determined according to the paddle method with sinker at 50, 75 or 100 rpm, preferably under *in vitro* conditions at 37 ±0.5 °C in 900 mL artificial gastric juice at pH 1.2, or under the same conditions in non-artificial gastric juice.

In a preferred embodiment, the pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, yet more preferably at least 80 wt.-%, most preferably at least 90 wt.-%, and in particular at least 95 wt.-% of the pharmacologically active agent according to general formula (I), based on the total amount of the pharmacologically active agent according to general formula (I) originally contained in the pharmaceutical dosage form.

The pharmaceutical dosage form according to the invention exhibits excellent shelf-life and storage stability, i.e. neither the chemical composition, nor the physical characteristics, nor the dissolution profile of the pharmaceutical dosage form are altered significantly upon storage.

In a preferred embodiment, the pharmaceutical dosage form according to the invention provides sufficient stability to the pharmacologically active agent according to general formula (I) contained therein, so that after storage of the pharmaceutical dosage form at 40±2°C at 75% RH ±5% for a minimum time period of 6 weeks, preferably 3 months, the concentrations of undesirable degradants and impurities, respectively, preferably resulting from a degradation or decomposition of the pharmacologically active agent according to general formula (I) as such, is at most 1.0 wt.-%, more preferably at most 0.8 wt.-%, still more preferably at most 0.6 wt.-%, yet more preferably at most 0.4 wt.-%, even more preferably at most 0.2 wt.-%, most preferably at most 0.1 wt.-%, and in particular at most 0.05 wt.-%, relative to the original content of the pharmacologically active agent according to general formula (I) in the pharmaceutical dosage form, i.e. its content before subjecting the pharmaceutical dosage form to storage.

It has been found that the pharmacologically active agent according to general formula (I) may be decomposed by elimination of the group -NRCH₃ thereby yielding 6'-fluoro-4-phenyl-4',9'-dihydro-3'H-spiro[cyclohex-3-ene-1,1'-pyrano[3,4-b]indole] which appears to be pharmacologically inactive. Preferably, after storage of the pharmaceutical dosage form at 40±2°C and 75% RH ±5%, or at 25±2°C and 60% RH ±5%, for a minimum time period of 6 weeks, preferably 3 months, the concentration of 6'-fluoro-4-phenyl-4',9'-dihydro-3'H-spiro[cyclohex-3-ene-1,1'-pyrano[3,4-b]indole] is at most 1.0 wt.-%, more preferably at most 0.8 wt.-%, still more preferably at most 0.6 wt.-%, yet more preferably at most 0.4 wt.-%, even more preferably at most 0.2 wt.-%, most preferably at most 0.1 wt.-%, and in particular at most 0.05 wt.-%, relative to the original content of the pharmacologically active agent according to general formula (I) in the pharmaceutical dosage form, i.e. its content before subjecting the pharmaceutical dosage form to storage.

A generally accepted accelerated test for the determination of a drug's stability according to ICH and FDA guidelines relates to the storage of a pharmaceutical formulation containing the drug (e.g., in its container and packaging). According to the ICH guidelines, a so-called accelerated storage testing should be conducted for pharmaceutical formulations at 40±2°C at 75% RH ±5% for a minimum time period of 6 months. Additionally, a so-called long-term storage testing should be conducted for pharmaceutical formulations at 25±2°C at not less than 60% RH ±5% for a minimum time period of 12 months. In case that all criteria have been met for the accelerated storage testing and long-term storage testing conditions during the 6-months period, the long-time storage testing may be shortened to 6 months and the corresponding data doubled to obtain estimated data for the 12-month period.

During the storage, samples of the pharmaceutical formulation are withdrawn at specified time intervals and analyzed in terms of their drug content, presence of impurities, their release profile and if applicable other parameters. According to the ICH guidelines, in all samples the purity of the drug should be ≥ 98%, the drug content should be 95-105% (FDA guideline: 90-110%). Furthermore, the pharmaceutical formulation should release >80% of the drug within 30 minutes.

In case of tablets and capsules that contain less than 50 mg of a drug, a content uniformity test should additionally be conducted for 10 randomly chosen dosage forms. The pharmaceutical formulation complies if none individual content is outside the limits of 85% to 115% of the average content. In case that an individual content is outside these limits, another 30 capsules have to be analyzed. The preparation fails to comply with the test if more than 3 individual contents are outside the limits of 85 to 115 % of the average content or if one or more individual contents are outside the limits of 75 % to 125 % of the average content.

In a preferred embodiment, after storage of the pharmaceutical dosage form for 6 months under long-term storage conditions (25°C and 60% relative humidity) in a sealed glass container, the degradation of the pharmacologically active agent according to general formula (I) does not exceed 2.0%, more preferably 1.5%, still more preferably 1.0%, and most preferably 0.5%.

In another preferred embodiment, after storage of the pharmaceutical dosage form for 6 months under accelerated storage conditions (40°C and 75% relative humidity) in a sealed glass container, the degradation of the pharmacologically active agent according to general formula (I) does not exceed 4%, more preferably 3%, still more preferably 2%, yet more preferably 1%, and most preferably 0.5%.

Preferably, after storage of the pharmaceutical dosage form for 6 months under long-term storage conditions (25°C and 60% relative humidity), the pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, and most preferably at least 80 wt.-% of the pharmacologically active agent according to general formula (I), based on the total amount of the pharmacologically active agent according to general formula (I) originally contained in the pharmaceutical dosage form.

Preferably, after storage of the pharmaceutical dosage form for 6 months under accelerated storage conditions (40°C and 75% relative humidity), the pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, and most preferably at least 80 wt.-% of the pharmacologically active agent according to general formula (I), based on the total amount of the pharmacologically active agent according to general formula (I) originally contained in the pharmaceutical dosage form.

The absorption properties of a pharmacologically active agent administered by a pharmaceutical dosage form can be described by the pharmacokinetic parameters Cₘₐₓ, tₘₐₓ and AUC₀₋ₜ. The determination of Cₘₐₓ and tₘₐₓ, as well as the calculation of an AUC are well known to a person skilled in the art and described, for example, in Bauer, Frömming, Führer, "Lehrbuch der Pharmazeutischen Technologie," 6th Edition (1999), and in Shargel, Wu-Pong, Yu, "Applied Biopharmaceuticals & Pharmacokinetics", 5th Edition (2005).

There is experimental evidence indicating that AUC₀₋ₜ and Cₘₐₓ of the pharmacologically active agent according to general formula (I) are proportional to the dose.

For the purpose of the specification, Cₘₐₓ is the highest plasma concentration of the pharmacologically active agent reached after single administration of the pharmaceutical dosage form.

For the purpose of the specification, tₘₐₓ is the time needed in order to reach Cₘₐₓ.

For the purpose of the specification, AUC₀₋ₜ is the area under the curve after single administration to the time t of the last sample that contained an analytically quantifiable concentration of the pharmacologically active agent.

For the purpose of the specification, AUC₀₋₇₂ₕ is the area under the curve baseline after single administration to 72 hours thereafter.

Preferably, the ratio Cₘₐₓ / dose is within the range of from 0.01 to 3.00 m⁻³, yet more preferably within the range of from 0.02 to 2.50 m⁻³, more preferably within the range of from 0.04 to 2.00 m⁻³, and most preferably within the range of from 0.06 to 1.69 m⁻³. In a preferred embodiment, the ratio Cₘₐₓ / dose is within the range of 0.40±0.35 m⁻³, more preferably 0.40±0.30 m⁻³, still more preferably 0.40±0.25 m⁻³, yet more preferably 0.40±0.20 m⁻³, even more preferably 0.40±0.15 m⁻³, most preferably 0.40±0.10 m⁻³, and in particular 0.40±0.05 m⁻³. In another preferred embodiment, the ratio Cₘₐₓ / dose is within the range of 0.80±0.70 m⁻³, more preferably 0.80±0.60 m⁻³, still more preferably 0.80±0.50 m⁻³, yet more preferably 0.80±0.40 m⁻³, even more preferably 0.80±0.30 m⁻³, most preferably 0.80±0.20 m⁻³, and in particular 0.80±0.10 m⁻³. In still another preferred embodiment, the ratio Cₘₐₓ / dose is within the range of 1.20±1.05 m⁻³, more preferably 1.20±0.90 m⁻³, still more preferably 1.20±0.75 m⁻³, yet more preferably 1.20±0.60 m⁻³, even more preferably 1.20±0.45 m⁻³, most preferably 1.20±0.30 m⁻³, and in particular 1.20±0.15 m⁻³.

Preferably, tₘₐₓ is within the range of from 15 minutes to 24 h, still more preferably within the range of from 20 minutes to 20 h, yet more preferably within the range of from 0.5 to 16 h, most preferably within the range of from 1 to 12 h, and in particular within the range of from 2 to 10 h. In a preferred embodiment, tₘₐₓ is within the range of 4±3.5 h, more preferably 4±3 h, still more preferably 4±2.5 h, yet more preferably 4±2 h, even more preferably 4±1.5 h, most preferably 4±1 h, and in particular 4±0.5 h. In another preferred embodiment, tₘₐₓ is within the range of 8±7 h, more preferably 8±6 h, still more preferably 8±5 h, yet more preferably 8±4 h, even more preferably 8±3 h, most preferably 8±2 h, and in particular 8±1 h. In still another preferred embodiment, tₘₐₓ is within the range of 12±11 h, more preferably 12±9 h, still more preferably 12±7 h, yet more preferably 12±5 h, even more preferably 12±3 h, most preferably 12±2 h, and in particular 12±1 h.

Preferably, the ratio AUC₀₋ₜ / dose is within the range from 0.3 to 20 h/m³, more preferably within the range of from 0.4 to 18 h/m³, still more preferably within the range of from 0.5 to 16.5 h/m³ and most preferably within the range of from 0.55 to 12.5 h/m³. In a preferred embodiment, the ratio AUC₀₋ₜ / dose is within the range of 3±2.5 h/m³, more preferably 3±2 h/m³, still more preferably 3±1.5 h/m³, yet more preferably 3±1 h/m³, even more preferably 3±0.75 h/m³, most preferably 3±0.5 h/m³, and in particular 3±0.25 h/m³. In another preferred embodiment, the ratio AUC₀₋ₜ / dose is within the range of 6±5 h/m³, more preferably 6±4 h/m³, still more preferably 6±3 h/m³, yet more preferably 6±2 h/m³, even more preferably 6±1.5 h/m³, most preferably 6±1 h/m³, and in particular 6±0.5 h/m³. In still another preferred embodiment, the ratio AUC₀₋ₜ / dose is within the range of 9±8 h/m³, more preferably 9±7 h/m³, still more preferably 9±5 h/m³, yet more preferably 9±4 h/m³, even more preferably 9±3 h/m³, most preferably 9±2 h/m³, and in particular 9±1 h/m³.

In a preferred embodiment, the pharmaceutical dosage form according to the invention is monolithic.

In another preferred embodiment, the pharmaceutical dosage form according to the invention comprises a core that is surrounded by a coating or by an encapsulating material. In a preferred embodiment, the core is liquid and the pharmacologically active agent according to general formula (I) is dispersed, preferably dissolved in the liquid.

Preferably, the pharmaceutical dosage form according to the invention provides the pharmacologically active agent according to general formula (I) in form of self-(micro) emulsifying drug delivery systems, solid solutions, nanoparticles, cyclodextrin complexes, liposomes, micelles, micronized and/or amorphous states.

In general terms, the options for formulation of poorly water-soluble drugs include crystalline solid, amorphous and lipid formulations.

The dissolution rate of the pharmacologically active agent from crystalline formulations can be increased by particle size reduction, thereby increasing the surface area for dissolution, e.g. by conventional micronisation of the the pharmacologically active agent to particle sizes of about 2-5 µm. In some cases, this is not sufficient and nanocrystal technology is applied. Nanocrystals show a particle size of 100-250 nm, which can be obtained by ball-milling or by dense gas or supercritical fluid technology.

Solid solutions provide and sustain the pharmacologically active agent in an amorphous state immobilized in a polymer. Amorphous solutions may contain surfactants and polymers, thereby providing surface-activity during dispersion upon contact with water. Solid solutions can be formed using a variety of technologies such as spray drying and melt extrusion.

Lipid formulations exhibiting different characteristics can be used to disperse and form micellar solutions, including simple solutions and self-emulsifying drug delivery systems (SEDDS). Depending on the excipients, some require digestion (e. g. simple oily liquids), others can easily be absorbed without digestion. Lipid formulations have been classified according to the lipid formulation classification system (LFCS) as follows:

| Excipients in formulation | Content of formulation (wt.-%) | | | | |
|---|---|---|---|---|---|
| | Type I | Type II | Type IIIA | Type IIIB | Type IV |
| Oil: triglycerides or mixed mono- and diglycerides | 100 | 40-80 | 40-80 | < 20 | - |
| Water-insoluble surfactants (HLB < 12) | - | 20-60 | - | - | 0-20 |
| Water-soluble surfactants (HLB > 12) | - | - | 20-40 | 20-50 | 30-80 |
| Hydrophilic co-solvent | - | - | 0-40 | 20-50 | 0-50 |

Another option is the formation of cyclodextrin complexes, in which the pharmacologically active agent is located in the cavity of the cyclodextrin and is thereby molecularly present in a more soluble form in presence of aqueous media. The success of the fitting strongly depends on the quality of the cyclodextrins as well as on the physicochemical properties and size of the pharmacologically active agent.

The pharmaceutical dosage form according to the invention can be regarded as a self emulsifying drug delivery system (SEDDS).

For that purpose, the pharmacologically active agent according to general formula (I) is preferably embedded in a self-emulsifying formulation. A so called self emulsifying drug delivery system (SEDDS) is a drug delivery system that uses an emulsion achieved by chemical rather than mechanical means. That is, by an intrinsic property of the drug formulation, rather than by special mixing and handling. Said formulation dilutes in aqueous media and results in an emulsion. In case that the average droplet size is smaller than or equal to 50 nm, the self emulsifying drug delivery system is referred to as self-micro emulsifying drug delivery system (SMEDDS). According to the lipid formulation classification system, these formulations are typically assigned to the group of type III formulations.

A preferred sub-group of SEDDSs are self-emulsifying oily formulations (SEOF). SEOFs typically comprise a natural or synthetic oil, surfactant and hydrophilic solvent and sometimes co-solvents. The principal characteristic of SEOFs is their ability to form fine oil-in-water emulsions or micro emulsions upon mild agitation following dilution by aqueous phases. These formulations can disperse in the gastrointestinal lumen to form micro emulsions or fine emulsions, upon dilution with gastrointestinal fluids.

In another preferred embodiment, the pharmaceutical dosage form contains the pharmacologically active agent according to general formula (I) in form of a solid solution, i.e. molecularly dispersed in a solid matrix. The solid solution preferably comprises the pharmacologically active agent according to general formula (I) in a molecular disperse form and an amorphous polymer matrix having a comparatively large specific surface. The pharmacologically active agent according to general formula (I) is preferably present in a molecular disperse form, i.e. the compound is truly solved and evenly spread in the solidified solution. The particle size of the compound is neither microcrystalline nor fine crystalline. The typical particle size is preferably from 0.1 - 1 µm.

In still another preferred embodiment, the pharmacologically active agent according to general formula (I) is provided by means of a nanotechnological formulation with an average size of the nanoparticles of preferably less than 1 µm. In a preferred embodiment, the pharmacologically active agent according to general formula (I) is provided in nanonized from. In another preferred embodiment, the pharmacologically active agent according to general formula (I) is blended with nanoparticles, preferably selected from organic nanoparticles and inorganic nanoparticles, and thus adsorbed to the surface of said particles.

Organic nanoparticles preferably contain small proteins which are present as a cluster or an agglomerate of small proteins, oligopeptides or lipids.

Inorganic nanoparticles preferably contain crystalline silicates. These silicates are from mineral origin or artificial silicates like metallosilicates (e.g. zeolites). In a preferred embodiment, the nanoparticles are modified in a way that they bear an electrostatic charge. The nanoparticles are preferably ultra finely grounded silicates and the pharmacologically active agent according to general formula (I) is preferably bounded to the micro porous surface of the nanoparticles.

The formation of nanoparticles is known to a person skilled in the art. One method is to produce colloidal nanoparticles as carriers for oral drug release by spraying the pharmacologically active agent according to general formula (I) under pressure at a defined temperature, together with a suitable carrier material like protamine, through jets, which are equipped with perforated strainers, into strongly cooled towers. The result of the fast cooling is an amorphous phase consisting of nanoparticles. Another method is to blend the pharmacologically active agent according to general formula (I) with suitable macromolecules in solution. By adding hydrophobic compounds, solvent molecules are removed from the solution and desolvation occurs. For this reason the formation of very tiny particles takes place wherein the pharmacologically active agent according to general formula (I) is integrated. For a hardening of the formed nanoparticles a crosslinker may be added to the solution.

To produce for example a solid lipid nanoparticle the method of high-pressure-homogenization and subsequent spray-cooling can be used. Preferably, the pharmacologically active agent according to general formula (I) is dissolved in a suitable solvent or in form of sub-micro particles. If applicable, a lipid vehicle and a surfactant may be added to the solution. Finally fine filler materials as outer phase as well as glidants and further surfactants may be added to fill the obtained formulation into e.g. capsules such as hard gelatin capsules.

In yet another preferred embodiment, the pharmacologically active agent according to general formula (I) are provided as cyclodextrin (inclusion) complexes.

Cyclodextrins are composed of sugar molecules forming a ring and typically comprising 5 or more α-D-glycopyranoside units which are linked via the 1-4 position. The typical number of connected sugar monomers ranges from 6 to 8 units. A six membered sugar ring molecule is called α-cyclodextrin. A seven membered sugar ring molecule is called β-cyclodextrin and an eight membered sugar ring molecule is called γ-cyclodextrin. The shape of these compounds is a toroid with the larger and the smaller openings exposed to the solvent. Due to this formation the inner part of the toroid is not hydrophobic, but considerably less hydrophilic than the aqueous environment and thus able to host hydrophobic molecules. The outer part of the toroid is sufficiently hydrophilic to render cyclodextrins water solubility.

The inclusion of the pharmacologically active ingredient according to general formula (I) in cyclodextrins greatly modifies the physical and chemical properties. In most cases the mechanism of controlled degradation of such complexes and resultant drug release is based on pH change of aqueous solutions, leading to the cleavage of hydrogen or ionic bonds between the cyclodextrins and the included molecules. Alternative means for the disruption of the complexes take advantage of heating or action of enzymes able to cleave α-1-4 linkages between α-D-glycopyranosides.

In another preferred embodiment, the pharmacologically active agent according to general formula (I) is provided in form of liposomes. A liposome is preferably composed of phospholipids and is preferably of spherical shape. The shell of this shape is preferably a lamellar or bilayer structure. Another type of phospholipids arrangement is a monolayer.

Phospholipids comprise molecules with an amphiphilic character i.e. the molecules have a hydrophobic (lipophilic) and a hydrophilic (lipophobic) part. In the presence of water, the hydrophilic part is attracted to the water and forms a surface facing to the water, while the hydrophobic part is repelled by the water and forms a surface away from the water. Hence the amphiphilic molecules arrange themselves in one of the mentioned types.

The bilayer structures preferably arrange in a spherical shape wherein the inner part is filled with an aqueous solution. This type is called "liposome". The hydrophobic parts of the molecules face each other in the middle of the layer and the hydrophilic parts of the molecules face the water molecules outside of the liposome. The aqueous solution inside the liposome is the same as it is outside of the liposome. Ingredients solved in this aqueous solution, e.g. the pharmacologically active agents according to general formula (I), are in this way inside of the liposome. A typical diameter of the liposomes is between 25 nm and 1 µm. The smaller ones (25 nm - 200 nm) are made of one single bilayer while the bigger ones (200 nm - 1 µm) comprise more bilayer shells on the top of each other.

The monolayer structures also arrange in spherical shapes. Due to the amphiphilic character of the molecules and the spherical shape of the monolayer structures, the inner part of the spherical structures is filled with/formed by the hydrophobic parts of the molecules. These types are called micelles. There is no solvent inside the structure. In a preferred embodiment, the inner parts of the micelles contain the pharmacologically active agents according to general formula (I).

In another preferred embodiment the pharmacologically active agent according to general formula (I) is provided in a micronized state. By means of micronization technique particles of the pharmacologically active agent according to general formula (I) with a diameter in nanometer scale can be prepared. Said particles have a large surface to volume ratio.

Milling and grinding is a useful method to obtain particles in nanometer scale. Sophisticated techniques for the micronization include RESS (rapid expansion of supercritical solutions), SAS (supercritical anti solvent) and the PGSS (particles from gas saturated solutions).

The RESS method uses a supercritical fluid wherein the pharmacologically active agent according to general formula (I) is dissolved under high pressure and temperature thereby yielding a homogenous supercritical phase. After expanding the solution through a nozzle, small particles are formed. Due to the expansion at the end of the nozzle the solved pharmacologically active agent according to general formula (I) precipitates as crystals and encloses small amounts of the solvent. The solvent changes from the supercritical fluid state to the normal state, preferred the gas phase, and breaks the crystals from inside-out. In this way and due to the fact that the crystals collide with each other, particles with a diameter in nanometer scale are formed.

In the SAS method the pharmacologically active agent according to general formula (I) is dissolved in a preferably organic solvent. A supercritical fluid is added to the solution under pressure and thus forced to also dissolve in the solvent. In consequence, the volume of the complete system is increased and the solubility of the pharmacologically active agent according to general formula (I) is decreased. Due to its decreased solubility, the compound according to general formula (I) precipitates and forms particles having a small diameter.

The PGSS method is similar to the SAS method. Here, the pharmacologically active agent according to general formula (I) is melted and a supercritical fluid is dissolved in the melt. Due to the expansion through a nozzle, the pharmacologically active agent according to general formula (I) precipitates and forms particles in a nanometer scale.

In a preferred embodiment, the pharmaceutical dosage form according to the invention contains
- a non-ionic surfactant (e.g. Cremophor^{®} EL, Cremophor^{®} RH 40, Cremophor^{®} RH 60, d-alpha-tocopherol polyethylene glycol 1000 succinate, polysorbate 20, polysorbate 80, Solutol^{®} HS 15, sorbitan monooleate, poloxamer 407, Labrafil^{®} M-1944CS, Labrafil^{®} M-2125CS, Labrasol^{®}, Gelucire^{®} 44/14, Softigen^{®} 767, and mono- and di-fatty acid esters of PEG 300, 400 or 1750); and/or
- an anionic surfactant such as sodium lauryl sulfate (sodium dodecyl sulfate, e.g. Texapon^{®} K12), sodium cetyl sulfate (e.g. Lanette E^{®}), sodium cetylstearyl sulfate, sodium stearyl sulfate, sodium dioctylsulfosuccinate (docusate sodium); and/or
- a water insoluble lipid (e.g. castor oil, corn oil cottonseed oil, olive oil, peanut oil, peppermint oil, safflower oil, sesame oil, soybean oil, hydrogenated vegetable oils, hydrogenated soybean oil, and medium chain triglycerides of coconut oil and palm seed oil); and/or
- an organic liquid/semi-solid (e.g. beeswax, d-alpha-tocopherol, oleic acid, medium chain mono- and diglycerides); and/or
- a cyclodextrin (e.g. alpha-cyclodextrin, beta-cyclodextrin, hydroxypropyl-beta-cyclodextrin, and sulfobutylether-beta-cyclodextrin); and/or
- a phospholipid (e.g. hydrogenated soy phosphatidylcholine, distearoylphosphatidylglycerol, L-alpha-dimyristoylphosphatidylcholine, and L-alpha-dimyristoylphosphatidylglycerol).

Preferably, the pharmacologically active agent according to general formula (I) is molecularly dispersed in a matrix.

In a preferred embodiment, the pharmacologically active agent according to general formula (I) is molecularly dispersed in a non-crystalline matrix.

In another preferred embodiment, the pharmacologically active agent according to general formula (I) is molecularly dispersed in a non-amorphous matrix.

Preferably, the pharmacologically active agent according to general formula (I) is homogeneously distributed in the pharmaceutical dosage form according to the invention. The content of the pharmacologically active agent according to general formula (I) of two segments of the pharmaceutical dosage form having a volume of 1.0 mm³ each, deviate from one another by preferably not more than ±10%, more preferably not more than more than ±7.5%, still more preferably not more than ±5.0%, most preferably not more than ±2.5%, and in particular not more than ±1.0%. When the pharmaceutical dosage form is encapsulated or film-coated, said two segments of the pharmaceutical dosage form having a volume of 1.0 mm³ each are preferably segments of the core, i.e. do not contain any encapsulating medium or film coating, respectively.

Preferably, the pharmaceutical dosage form according to the invention is characterized by a comparatively homogeneous distribution of density. Preferably, the densities of two segments of the pharmaceutical dosage form having a volume of 1.0 mm³ each, deviate from one another by not more than ±10%, more preferably not more than more than ±7.5%, still more preferably not more than ±5.0%, most preferably not more than ±2.5%, and in particular not more than ±1.0%. When the pharmaceutical dosage form is encapsulated, said two segments of the pharmaceutical dosage form having a volume of 1.0 mm³ each are preferably segments of the core, i.e. do not contain any encapsulating medium or film coating.

In a preferred embodiment, the pharmaceutical dosage form further contains a surfactant.

For the purpose of the specification, the term "surfactant" refers to any compound that contains at least one hydrophobic group and at least one hydrophilic group. Preferably, the surfactant contains at least one terminal hydrophobic group (tail) and at least one terminal hydrophilic group (head).

The hydrophobic group is preferably selected from the group consisting of hydrocarbon, alkyl ether, fluorocarbon and siloxan groups.

In a preferred embodiment, the surfactant contains at least one aliphatic group comprising at least 3 carbon atoms, more preferably at least 4 carbon atoms, still more preferably at least 6 carbon atoms, yet more preferably 6 to 30 carbon atoms, and most preferably 8 to 24 carbon atoms. The aliphatic group may be a saturated or unsaturated, branched or unbranched (linear), terminal or internal aliphatic group.

Preferably, the surfactant contains at least one group derivable from a saturated or unsaturated fatty acid or from a saturated or unsaturated fatty alcohol, which group is preferably an ether, carboxylic acid ester or sulfuric acid ester group. Preferably, the saturated or unsaturated fatty acid or fatty alcohol contains at least 6 carbon atoms, yet more preferably 6 to 30 carbon atoms, and most preferably 8 to 24 carbon atoms.

In a preferred embodiment, the surfactant contains at least one group derivable from a saturated or unsaturated fatty acid, preferably C₆ to C₃₀ fatty acid, more preferably C₈ to C₂₄ fatty acid, and most preferably C₁₂ to C₂₂ fatty acid. Examples for suitable fatty acids are lauric acid, myristic acid, palmitic acid, stearic acid, arachidic acid, behenic acid, lignoceric acid, 12-hydroxystearic acid, oleic acid and ricinoleic acid.

In another preferred embodiment, the surfactant contains at least one group derivable from a saturated or unsaturated fatty alcohol, preferably C₆ to C₃₀ fatty alcohol, more preferably C₈ to C₂₄ fatty alcohol, and most preferably C₁₂ to C₂₂ fatty alcohol. Examples for suitable fatty alcohols are cetyl alcohol, stearyl alcohol, 2-octyldodecane-1-ol and 2-hexyldecane-1-ol.

Preferably, the surfactant has a molecular weight of at most 20,000 g/mol, more preferably at most 15,000 g/mol, still more preferably at most 10,000 g/mol, yet more preferably at most 5,000 g/mol, even more preferably at most 4,000 g/mol, most preferably at most 3,000 g/mol, and in particular within the range of from 100 g/mol to 2,500 g/mol.

Preferably, the surfactant is contained in a matrix in which the pharmacologically active agent according to general formula (I) is dispersed, preferably molecularly.

In a preferred embodiment, the pharmacologically active agent according to general formula (I) and the surfactant are intimately homogeneously distributed in a matrix so that the matrix does not contain any segments where either the pharmacologically active agent according to general formula (I) is present in the absence of the surfactant or where the surfactant is present in the absence of the pharmacologically active agent according to general formula (I).

In a preferred embodiment, the pharmaceutical dosage form contains a surfactant. In another preferred embodiment, the pharmaceutical dosage form contains a mixture of two or more surfactants.

In a preferred embodiment, the surfactant acts as an O/W emulsifier. In another preferred embodiment, the surfactant acts as a W/O emulsifier.

Preferably, the pharmaceutical dosage form contains a surfactant having a hydrophilic-lipophilic balance (HLB) of at least 10 or at least 11. More preferably, the hydrophilic-lipophilic balance (HLB) is at least 12 or at least 13. Most preferably, the hydrophilic-lipophilic balance (HLB) ranges within 14 and 16.

Preferably, the hydrophilic-lipophilic balance (HLB) of the surfactant is at most 30, more preferably at most 28, still more preferably at most 26, yet more preferably at most 24, even more preferably at most 22, most preferably at most 20 and in particular at most 18.

In another preferred embodiment, the hydrophilic-lipophilic balance (HLB) of the surfactant is at least 27, more preferably at least 29, still more preferably at least 31, yet more preferably at least 33, even more preferably at least 35, most preferably at least 37 and in particular at least 39.

In a preferred embodiment, the HLB value of the surfactant is within the range of 10±3.5, more preferably 10±3, still more preferably 10±2.5, yet more preferably 10±2, even more preferably 10±1.5, most preferably 10±1, and in particular 10±0.5. In another preferred embodiment, the HLB value of the surfactant is within the range of 12±3.5, more preferably 12±3, still more preferably 12±2.5, yet more preferably 12±2, even more preferably 12±1.5, most preferably 12±1, and in particular 12±0.5. In still another preferred embodiment, the HLB value of the surfactant is within the range of 14±3.5, more preferably 14±3, still more preferably 14±2.5, yet more preferably 14±2, even more preferably 14±1.5, most preferably 14±1, and in particular 14±0.5. In another preferred embodiment, the HLB value of the surfactant is within the range of 15±3.5, more preferably 15±3, still more preferably 15±2.5, yet more preferably 15±2, even more preferably 15±1.5, most preferably 15±1, and in particular 15±0.5. In yet another preferred embodiment, the HLB value of the surfactant is within the range of 16±3.5, more preferably 16±3, still more preferably 16±2.5, yet more preferably 16±2, even more preferably 16±1.5, most preferably 16±1, and in particular 16±0.5. In another preferred embodiment, the HLB value of the surfactant is within the range of 18±3.5, more preferably 18±3, still more preferably 18±2.5, yet more preferably 18±2, even more preferably 18±1.5, most preferably 18±1, and in particular 18±0.5.

The surfactant can be ionic, amphoteric or non-ionic.

In a preferred embodiment, the pharmaceutical dosage form contains an ionic surfactant, in particular an anionic surfactant.

Suitable anionic surfactants include but are not limited to sulfuric acid esters such as sodium lauryl sulfate (sodium dodecyl sulfate, e.g. Texapon^{®} K12), sodium cetyl sulfate (e.g. Lanette E^{®}), sodium cetylstearyl sulfate, sodium stearyl sulfate, sodium dioctylsulfosuccinate (docusate sodium); and the corresponding potassium or calcium salts thereof.

Preferably, the anionic surfactant has the general formula (II-a)

CₙH₂ₙ₊₁O-SO₃⁻ M⁺ (II-a),

wherein n is an integer of from 8 to 30, preferably 10 to 24, more preferably 12 to 18; and M is selected from Li⁺, Na⁺, K⁺, NH₄⁺ 1/2 Mg²⁺ and 1/2 Ca²⁺.

Further suitable anionic surfactants include salts of cholic acid including sodium glycocholate (e.g. Konakion^{®} MM, Cernevit^{®}), sodium taurocholate and the corresponding potassium or ammonium salts.

In another preferred embodiment, the pharmaceutical dosage form contains a non-ionic surfactant. Suitable non-ionic surfactants include but are not limited to
- fatty alcohols that may be linear or branched, such as cetylalcohol, stearylalcohol, cetylstearyl alcohol, 2-octyldodecane-1-ol and 2-hexyldecane-1-ol;
- sterols, such as cholesterole;
- partial fatty acid esters of sorbitan such as sorbitanmonolaurate, sorbitanmonopalmitate, sorbitanmonostearate, sorbitantristearate, sorbitanmonooleate, sorbitansesquioleate and sorbitantrioleate;
- partial fatty acid esters of polyoxyethylene sorbitan (polyoxyethylene-sorbitan-fatty acid esters), preferably a fatty acid monoester of polyoxyethylene sorbitan, a fatty acid diester of polyoxyethylene sorbitan, or a fatty acid triester of polyoxyethylene sorbitan; e.g. mono-and tri- lauryl, palmityl, stearyl and oleyl esters, such as the type known under the name "polysorbat" and commercially available under the trade name "Tween" including Tween^{®} 20 [polyoxyethylene(20)sorbitan monolaurate], Tween^{®} 21 [polyoxyethylene(4)sorbitan monolaurate], Tween^{®} 40 [polyoxyethylene(20)sorbitan monopalmitate], Tween^{®} 60 [polyoxyethylene(20)sorbitan monostearate], Tween^{®} 65 [polyoxyethylene(20)sorbitan tristearate], Tween^{®} 80 [polyoxyethylene(20)sorbitan monooleate], Tween 81 [polyoxyethylene(5)sorbitan monooleate], and Tween^{®} 85 [polyoxyethylene(20)sorbitan trioleate]; preferably a fatty acid monoester of polyoxyethylenesorbitan according to general formula (II-b) 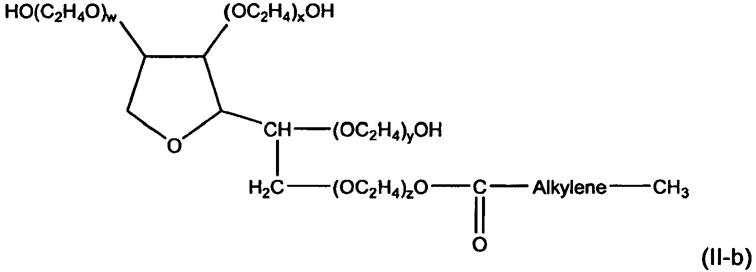 wherein (w+x+y+z) is within the range of from 15 to 100, preferably 16 to 80, more preferably 17 to 60, still more preferably 18 to 40 and most preferably 19 to 21;
   and alkylene is an optionally unsaturated alkylene group comprising 6 to 30 carbon atoms, more preferably 8 to 24 carbon atoms and most preferably 10 to 16 carbon atoms;
- polyoxyethyleneglycerole fatty acid esters such as mixtures of mono-, di- and triesters of glycerol and di- and monoesters of macrogols having molecular weights within the range of from 200 to 4000 g/mol, e.g., macrogolglycerolcaprylocaprate, macrogolglycerollaurate, macrogolglycerolococoate, macrogolglycerollinoleate, macrogol-20-glycerolmonostearate, macrogol-6-glycerolcaprylocaprate, macrogolglycerololeate; macrogolglycerolstearate, macrogolglycerolhydroxystearate (e.g. Cremophor^{®} RH 40), and macrogolglycerolrizinoleate (e.g. Cremophor^{®} EL);
- polyoxyethylene fatty acid esters, the fatty acid preferably having from about 8 to about 18 carbon atoms, e.g. macrogololeate, macrogolstearate, macrogol-15-hydroxystearate, polyoxyethylene esters of 12-hydroxystearic acid, such as the type known and commercially available under the trade name "Solutol HS 15"; preferably according to general formula (II-c)

   CH₃CH₂-(OCH₂CH₃)ₙ-O-CO-(CH₂)ₘCH₃ (II-c)

   wherein n is an integer of from 6 to 500, preferably 7 to 250, more preferably 8 to 100, still more preferably 9 to 75, yet more preferably 10 to 50, even more preferably 11 to 30, most preferably 12 to 25, and in particular 13 to 20; and
   wherein m is an integer of from 6 to 28; more preferably 6 to 26, still more preferably 8 to 24, yet more preferably 10 to 22, even more preferably 12 to 20, most preferably 14 to 18 and in particular 16;
- polyoxyethylene fatty alcohol ethers, e.g. macrogolcetylstearylether, macrogollarylether, macrogololeylether, macrogolstearylether;
- polyoxypropylene-polyoxyethylene block copolymers (poloxamers);
- fatty acid esters of saccharose; e.g. saccharose distearate, saccharose dioleate, saccharose dipalmitate, saccharose monostearate, saccharose monooleate, saccharose monopalmitate, saccharose monomyristate and saccharose monolaurate;
- fatty acid esters of polyglycerol, e.g. polyglycerololeate;
- polyoxyethylene esters of alpha-tocopheryl succinate, e.g. D-alpha-tocopheryl-PEG-1000-succinate (TPGS);
- polyglycolyzed glycerides, such as the types known and commercially available under the trade names "Gelucire 44/14", "Gelucire 50/13 and "Labrasol";
- reaction products of a natural or hydrogenated castor oil and ethylene oxide such as the various liquid surfactants known and commercially available under the trade name "Cremophor"; and
- partial fatty acid esters of multifunctional alcohols, such as glycerol fatty acid esters, e.g. mono- and tri-lauryl, palmityl, stearyl and oleyl esters, for example glycerol monostearate, glycerol monooleate, e.g. glyceryl monooleate 40, known and commercially available under the trade name "Peceol"; glycerole dibehenate, glycerole distearate, glycerole monolinoleate; ethyleneglycol monostearate, ethyleneglycol monopalmitostearate, pentaerythritol monostearate.

In a particularly preferred embodiment, the pharmaceutical dosage form according to the invention comprises a surfactant or mixture of different surfactants obtainable by
(i) esterifying saturated or unsaturated C₁₂-C₁₈-fatty acids, optionally bearing a hydroxyl group, with a polyethylene glycol and optionally, glycerol; wherein the polyethylene glycol preferably comprises 10 to 40 ethylene oxide units (-CH₂CH₂O-); and/or
(ii) etherifying triglycerides of saturated or unsaturated C₁₂-C₁₈-fatty acids bearing a hydroxyl group with ethylene oxide so that a polyethylene glycol moiety is linked to the hydroxyl group of the C₁₂-C₁₈-fatty acids via an ether bond; wherein the polyethylene glycol moiety preferably comprises 30 to 50 ethylene oxide units (-CH₂CH₂O-).

Preferably, the surfactant is selected from the group consisting of macrogolhydroxystearate, macrogolglycerylhydroxystearate and macrogolglyceryllaurate, wherein the macrogol moiety preferably comprises 15 to 45 ethylene oxide units.

Especially preferred surfactants of this class that are contained in the pharmaceutical dosage form according to the invention are non-ionic surfactants having a hydrophilic-lipophilic balance (HLB) of at least 10, in particular non-ionic surfactants having an HLB value of at least 12, more in particular non-ionic surfactant's having an HLB value within 14 and 16. Examples for this type of surfactants are the above-listed surfactants "Tween^{®} 80" and "Solutol^{®} HS 15".

Solutol^{®} HS-15 is a mixture of polyethyleneglycol 660 12-hydroxystearate and polyethylene glycol. It is a white paste at room temperature that becomes liquid at about 30°C and has an HLB of about 15.

Tween^{®} 80 [polyoxyethylene(20)sorbitan monooleate] is liquid at room temperature, has a viscosity of 375-480 mPa·s and has an HLB of about 15.

In another preferred embodiment the pharmaceutical dosage form according to the invention contains a mixture of at least one surfactant having a HLB value of at least 10 (hydrophilic surfactant) and at least one surfactant having a HLB value below 10 (lipophilic surfactant). For example, the dosage form may contain macrogol-glycerolhydroxystearat 40 (e.g., Cremophor^{®} RH 40) as the hydrophilic surfactant component and glyceryl monooleate 40 (e.g., Peceol^{®}) as the lipophilic surfactant component.

Preferably, the relative weight ratio of the surfactant having a HLB value of at least 10 (hydrophilic surfactant) and the surfactant having a HLB value below 10 (lipophilic surfactant) is within the range of 15:1 to 1:20, more preferably 10:1 to 1:15, still more preferably 8:1 to 1:12, yet more preferably 6:1 to 1:10, even more preferably 5:1 to 1:7, most preferably 4:1 to 1:4 and in particular 2:1 to 1:2.

In a preferred embodiment, the content of the surfactant is at least 0.001 wt.-% or at least 0.005 wt.-%, more preferably at least 0.01 wt.-% or at least 0.05 wt.-%, still more preferably at least 0.1 wt.-%, at least 0.2 wt.-%, or at least 0.3 wt.-%, yet more preferably at least 0.4 wt.-%, at least 0.5 wt.-%, or at least 0.6 wt.-%, and in particular at least 0.7 wt.-%, at least 0.8 wt.-%, at least 0.9 wt.-%, or at least 1.0 wt.-%, based on the total weight of the pharmaceutical dosage form.

In another preferred embodiment, particularly when the pharmaceutical dosage form contains an encapsulated core, the content of the surfactant is at least 10 wt.-%, more preferably at least 15 wt.-%, still more preferably at least 20 wt.-%, yet more preferably at least 25 wt.-% and in particular at least 30 wt.-%, based on the total weight of the composition forming the core. In a preferred embodiment, the content of the surfactant ranges preferably from 0.1 wt.-% to 95 wt.-%, more preferably from 1 wt.-% to 95 wt.-%, still more preferably from 5 wt.-% to 90 wt.-%, yet more preferably from 10 wt.-% to 80 wt.-%, most preferably from 20 wt.-% to 70 wt.-%, and in particular from 30 wt.-% to 75 wt.-%, based on the total weight of the composition forming the core.

In a preferred embodiment, the pharmaceutical dosage form contains a core that is encapsulated by an encapsulating medium. The core can be liquid, semi-liquid or solid.

Preferably, said encapsulating medium is a soft gelatin capsule or a hard gelatin capsule, in particular a hard gelatin capsule.

In an especially preferred embodiment, the pharmaceutical dosage form comprises a liquid core encapsulated by a solid material, wherein the pharmacologically active agent according to general formula (I) is dispersed in the liquid core. Preferably, the solid material is a hard gelatin capsule.

The pharmaceutical dosage form according to the invention contains a self-emulsifying formulation in which the pharmacologically active agent according to general formula (I) is preferably embedded. Preferably, the pharmacologically active agent according to general formula (I) is molecularly dispersed in the other ingredients of liquid core. For the purpose of the specification, "molecularly dispersed in a liquid core", e.g. in the other ingredients of the liquid core, means that a substantial portion of the overall content of the pharmacologically active agent according to general formula (I) is present in non-crystalline form, i.e. does not provide X-ray reflexes. Preferably, the pharmacologically active agent according to general formula (I) is dissolved in the other ingredients of the core. Preferably, the content of non-crystalline pharmacologically active agent according to general formula (I) is at least 60 wt.-%, more preferably at least 65 wt.-%, still more preferably at least 70 wt.-%, yet more preferably at least 75 wt.-%, even more preferably at least 80 wt.-%, most preferably at least 85 wt.-%, and in particular at least 90 wt.-%, based on the total content of pharmacologically active agent according to general formula (I).

In a preferred embodiment, the self-emulsifying formulation contains the surfactant and an oil.

In another preferred embodiment, the self-emulsifying formulation is a self-emulsifying oily formulation (SEOF), i.e. it comprises the surfactant, the oil and additionally a hydrophilic solvent.

For the purpose of the specification, an oil is preferably to be regarded as any substance that is liquid at ambient temperatures or has a melting point below 70 °C and is hydrophobic but soluble in organic solvents.

Preferably, the oil is a C₁₂-C₁₈-fatty acid ester of a monoalcohol (e.g. C₁-C₁₂-alkylalcohols), a di-C₁₂-C₁₈-fatty acid ester of a dialcohol (e.g. ethylene glycol) or tri-C₁₂-C₁₈-fatty acid ester of a trialcohol (e.g. glycerol).

Preferably, the oil has a melting point below 60°C, more preferably below 55°C, still more preferably below 50°C, yet more preferably below 45°C, even more preferably below 40°C, most preferably below 35°C and in particular below 30°C.

Preferably, the pure oil has a density within the range of 0.94±0.07 g/cm³, more preferably 0.94±0.06 g/cm³, still more preferably 0.94±0.05 g/cm³, yet more preferably 0.94±0.04 g/cm³, even more preferably 0.94±0.03 g/cm³, most preferably 0.94±0.02 g/cm³, and in particular 0.94±0.01 g/cm³.

Preferably, the pure oil has a viscosity at 20°C measured in accordance with Ph.Eur. 2.2.8, within the range of 30±9 mPas, more preferably 30±8 mPas, still more preferably 30±7 mPas, yet more preferably 30±6 mPas, even more preferably 30±5 mPas, most preferably 30±4 mPas, and in particular 30±3 mPas.

In a preferred embodiment, the oil is selected from the group consisting of
- saturated C₈ to C₁₄ fatty acids, such as myristic acid;
- unsaturated C₈ to C₁₈ fatty acids and their esters, such as oleic acid and ethyl oleate;
- mixtures of saturated and unsaturated C₈ to C₁₈ fatty acids, such as soybean oil and peanut oil; and
- triglycerides of fatty acids, preferably of C₆ to C₁₂ fatty acids, more preferably of C₆ to C₁₀ fatty acids, such as the caprylic/capric triglyceride mixtures, most preferably medium-chain triglycerides according to Ph. Eur. or USP, e.g. known and commercially available under the trade names "Captex 355" and Miglyol 812"; and
- propylene glycol fatty acid esters such as propylene glycol monocaprylate (known and commercially available under the trade names "Capryol 90");
especially preferred are medium-chain triglycerides according to Ph. Eur. or USP such as said caprylic/capric triglyceride mixtures.

In a preferred embodiment, the content of the oil in the pharmaceutical dosage form is within the range of from 1 wt.-% to 90 wt.-%, preferably from 2 wt.-% to 80 wt.-%, more preferably from 5 wt.-% to 60 wt.-%, still more preferably from 10 wt.-% to 50 wt.-% and most preferably from 15 wt.-% to 30 wt.-%, preferably based on the total weight of the core.

In a preferred embodiment, the relative weight ratio of the surfactant to the oil is within the range of from 20:1 to 1:20, more preferably 10:1 to 1:10, still more preferably 7.5:1 to 1:5, yet more preferably 7:1 to 1:1, most preferably 5:1 to 1.5:1 and in particular 4:1 to 2:1.

Preferably, the self-emulsifying formulation is present as the liquid core, encapsulated by a hard gelatin capsule.

In a preferred embodiment, the self-emulsifying formulation further contains a hydrophilic solvent.

Preferably, the hydrophilic solvent is an organic alcohol such as an organic monoalcohol, organic dialcohol or organic trialcohol.

Preferably, the pure hydrophilic solvent has a boiling point at ambient pressure within the range of 78±22°C, more preferably 78±18°C, still more preferably 78±15°C, yet more preferably 78±12°C, even more preferably 78±8°C, most preferably 78±5°C, and in particular 78±2°C.

Preferably, the hydrophilic solvent is selected from the group ethanol, isopropanol, glycerol and propylene glycol; especially preferred is ethanol. Preferably, the content of the hydrophilic solvent is within the range of from about 1 wt.-% to about 90 wt.-%, preferably from about 2 wt.-% to about 80 wt.-%, more preferably from about 5 wt.-% to about 60 wt.-%, still more preferably from about 10 wt.-% to about 50 wt.-%, most preferably from about 15 wt.-% to about 30 wt.-%, preferably based on the total weight of the core.

In a preferred embodiment, the pharmaceutical dosage form contains a liquid core comprising the pharmacologically active agent according to general formula (I), a surfactant, an oil and a hydrophilic solvent, wherein the relative weight ratio of surfactant : oil : hydrophilic solvent is within the range of 60 : 20±17.5 : 20±17.5, more preferably 60 : 20±15 : 20±15, still more preferably 60 : 20±12.5 : 20±12.5, yet more preferably 60 : 20±10 : 20±10, even more preferably 60 : 20±7.5 : 20±7.5, most preferably 60 : 20±5 : 20±5, and in particular 60 : 20±2.5 : 20±2.5.

In another preferred embodiment, the pharmaceutical dosage form contains a liquid core comprising the pharmacologically active agent according to general formula (I), a surfactant having a HLB value of at least 10 (hydrophilic surfactant), an oil and a surfactant having a HLB value below 10 (lipophilic surfactant), wherein the relative weight ratio of hydrophilic : oil : lipophilic solvent is within the range of 60 : 20±17.5 : 20±17.5, more preferably 60 : 20±15 : 20±15, still more preferably 60 : 20±12.5 : 20±12.5, yet more preferably 60 : 20±10 : 20±10, even more preferably 60 : 20±7.5 : 20±7.5, most preferably 60 : 20±5 : 20±5, and in particular 60 : 20±2.5 : 20±2.5.

In another preferred embodiment, the pharmaceutical dosage form contains a liquid core comprising the pharmacologically active agent according to general formula (I), a surfactant having a HLB value of at least 10 (hydrophilic surfactant), an oil and a surfactant having a HLB value below 10 (lipophilic surfactant), wherein the relative weight ratio of hydrophilic : oil : lipophilic solvent is within the range of 40 : 40±35 : 20±17.5, more preferably 40 : 40±30 : 20±15, still more preferably 40 : 40±25 : 20±12.5, yet more preferably 40 : 40±20 : 20±10, even more preferably 40 : 40±15 : 20±7.5, most preferably 40 : 40±10 : 20±5, and in particular 40 : 40±5 : 20±2.5.

Preferred embodiments A¹ to A²⁰ of the liquid core of the pharmaceutical dosage form according to the invention, i.e. of the liquid core that is encapsulated by an encapsulating material, are summarized in the table here below:

| embodiment | A¹ | | A² | | A³ | | A⁴ | |
|---|---|---|---|---|---|---|---|---|
| inqredient | nature | cont. | nature | cont. | nature | cont. | nature | cont. |
| pharmacologically active agent according to general formula (I) | W¹ | 0.50±0.49 | W¹ | 0.50±0.49 | W¹ | 0.50±0.49 | W¹ | 0.50±0.49 |
| surfactant | X¹ | 30±25 | X¹ | 45±30 | X¹ | 60±40 | X¹ | 60±40 |
| oil | Y¹ | 40±35 | Y¹ | 40±30 | Y¹ | 15±10 | Y¹ | 25±20 |
| additional component | Z¹ | 30±25 | Z¹ | 15±10 | Z¹ | 25±20 | Z¹ | 15±10 |

| embodiment | A⁵ | | A⁶ | | A⁷ | | A⁸ | |
|---|---|---|---|---|---|---|---|---|
| ingredient | nature | cont. | nature | cont. | nature | cont. | nature | cont. |
| pharmacologically active agent according to general formula (I) | W¹ | 0.50±0.49 | W¹ | 0.25±0.24 | W² | 0.25±0.24 | W² | 0.25±0.24 |
| surfactant | X¹ | 60±40 | X² | 40±15 | X² | 60±20 | X² | 60±20 |
| oil | Y¹ | 20±15 | Y² | 30±15 | Y² | 10±5 | Y² | 30±15 |
| additional component | Z¹ | 20±15 | Z² | 30±15 | Z² | 30±15 | Z² | 10±5 |

| embodiment | A⁹ | | A¹⁰ | | A¹¹ | | A¹² | |
|---|---|---|---|---|---|---|---|---|
| ingredient | nature | cont. | nature | cont. | nature | cont. | nature | cont. |
| pharmacologically active agent according to general formula (I) | W² | 0.25±0.24 | W² | 0.25±0.24 | W² | 0.10±0.09 | W² | 0.10±0.09 |
| surfactant | X² | 50±15 | X² | 60±15 | X³ | 40±10 | X³ | 50±10 |
| oil | Y² | 25±7.5 | Y² | 20±7.5 | Y³ | 30±10 | Y³ | 30±10 |
| additional component | Z² | 25±7.5 | Z² | 20±7.5 | Z³ | 30±10 | Z³ | 20±7.5 |

| embodiment | A¹³ | | A¹⁴ | | A¹⁵ | | A¹⁶ | |
|---|---|---|---|---|---|---|---|---|
| ingredient | nature | cont. | nature | cont. | nature | cont. | nature | cont. |
| pharmacologically active agent according to general formula (I) | W² | 0.10±0.09 | W³ | 0.10±0.09 | W³ | 0.10±0.05 | W³ | 0.02±0.01 |
| surfactant | X³ | 60±10 | X³ | 70±10 | X⁴ | 50±5 | X⁴ | 50±5 |
| oil | Y³ | 20±10 | Y³ | 15±5 | Y⁴ | 25±2.5 | Y⁴ | 25±2.5 |
| additional component | Z³ | 20±20 | Z³ | 15±5 | Z⁴ | 25±2.5 | Z⁴ | 25±2.5 |

| embodiment | A¹⁷ | | A¹⁸ | | A¹⁹ | | A²⁰ | |
|---|---|---|---|---|---|---|---|---|
| ingredient | nature | cont. | nature | cont. | nature | cont. | nature | cont. |
| pharmacologically active agent according to general formula (I) | W³ | 0.10±0.05 | W³ | 0.02±0.01 | W³ | 0.10±0.05 | W³ | 0.02±0.01 |
| surfactant | X⁴ | 60±5 | X⁴ | 60±5 | X⁴ | 60±5 | X⁴ | 60±5 |
| oil | Y⁴ | 25±2.5 | Y⁴ | 25±2.5 | Y⁴ | 20±2.5 | Y⁴ | 20±2.5 |
| additional component | Z⁴ | 15±2.5 | Z⁴ | 15±2.5 | Z⁴ | 20±2.5 | Z⁴ | 20±2.5 |

wherein
nature refers to the chemical nature of the ingredient;
cont. refers to the content of the ingredient in wt.-% based on the total weight of the core;
- W¹: means pharmacologically active agent according to general formula (I) or a physiologically acceptable salt thereof;
- W²: means pharmacologically active agent according to general formula (I') or a physiologically acceptable salt thereof;
- W³: means (1r,4r)-6'-fluoro-N,N-dimethyl-4-phenyl-4',9'-dihydro-3'H-spiro[cyclohexane-1,1'-pyrano[3,4,b]indol]-4-amine, or (1r,4r)-6'-fluoro-N-methyl-4-phenyl-4',9'-dihydro-3'H-spiro[cyclohexane-1,1'-pyrano[3,4,b]indol]-4-amine, or a physiologically acceptable salt thereof;
- X¹: means surfactant having a HLB value of at least 10;
- X²: means non-ionic surfactant having a HLB value of between 14 and 16;
- X³: means polyglycolyzed glyceride;
- X⁴: means polyoxyethylene fatty acid ester, the fatty acid preferably having from about 8 to about 18 carbon atoms;
- Y¹: means mono-, di- or triester of the C₆ to C₁₈ fatty acids;
- Y²: means triglycerides of C₆ to C₁₂ fatty acids (medium-chain triglycerides);
- Y³: means propylene glycol fatty acid ester;
- Y⁴: caprylic/capric triglyceride mixture;
- Z¹: means hydrophilic solvent
- Z²: means hydrophilic solvent selected from organic monoalcohol, dialcohol or trialcohol;
- Z³: means surfactant having a HLB value of below 10;
- Z⁴: means ethanol.

For example, according to the above table, embodiment A⁹ relates to a pharmaceutical dosage according to the invention, which contains a pharmacologically active agent according to general formula (I') or a physiologically acceptable salt thereof in an amount of 0.25±0.24 wt.-%, a non-ionic surfactant having a HLB value of between 14 and 16 in an amount of 50t15 wt.-%, triglycerides of the C₆ to C₁₂ fatty acids in an amount of 25±7.5% and a hydrophilic solvent selected from organic monoalcohol, dialcohol or trialcohol in an amount of 25±7.5%, based on the total weight of the liquid core.

Preferably, the self-emulsifying formulation is a lipid formulation of type IIIA or type IIIB, according to the lipid formulation classification system (LFCS).

Preferably, the self emulsifying formulation gives emulsions with an average droplet size smaller than or equal to 10 micrometers, more preferably smaller than or equal to 1000 nanometers, most preferably smaller than or equal to 100 nanometers, when exposed to aqueous media.

In another preferred embodiment, the self-emulsifying formulation is a self-micro emulsifying drug delivery system (SMEDDS), i.e. when exposed to aqueous media, the formulation gives microemulsions with an average droplet size smaller than or equal to 50 nanometers, which contain the pharmacologically active agent according to general formula (I). In another preferred embodiment, the average droplet size is smaller than or equal to 10 nanometers

In a preferred embodiment, the average droplet size is within the range of 50±70 nm, more preferably 50±60 nm, still more preferably 50±50 nm, yet more preferably 50±40 nm, even more preferably 50±30 nm, most preferably 50±20 nm, and in particular 50±10 nm.

In a preferred embodiment, the average droplet size is within the range of 75±70 nm, more preferably 75±60 nm, still more preferably 75±50 nm, yet more preferably 75±40 nm, even more preferably 75±30 nm, most preferably 75±20 nm, and in particular 75±10 nm.

In a preferred embodiment, the average droplet size is within the range of 100±70 nm, more preferably 100±60 nm, still more preferably 100±50 nm, yet more preferably 100±40 nm, even more preferably 100±30 nm, most preferably 100±20 nm, and in particular 100±10 nm.

In a preferred embodiment, the average droplet size is within the range of 125±70 nm, more preferably 125±60 nm, still more preferably 125±50 nm, yet more preferably 125±40 nm, even more preferably 125±30 nm, most preferably 125±20 nm, and in particular 125±10 nm.

In a preferred embodiment, the average droplet size is within the range of 150±70 nm, more preferably 150±60 nm, still more preferably 150±50 nm, yet more preferably 150±40 nm, even more preferably 150±30 nm, most preferably 150±20 nm, and in particular 150±10 nm.

In a particular preferred embodiment,
- the pharmaceutical dosage form contains a surfactant having a HLB value of at least 10 in an amount of at least 0.001 wt.-%, based on the total weight of the pharmaceutical dosage form; and/or
- the pharmaceutical dosage form contains 0.01 % to 95 % of the pharmacologically active agent (A); and/or
- the pharmaceutical dosage form has a weight within the range of from 0.1 mg to 2,000 mg; and/or
- the pharmaceutical dosage form contains a polymer with a molecular weight within the range of from 1,000 g/mol to 15 million g/mol; and/or
- the pharmaceutical dosage form is for oral administration; and/or
- the pharmaceutical dosage form contains the pharmacologically active agent according to general formula (I) in a dose of from 10 µg to 50 µg or of from 300 µg to 500 µg; and/or
- the pharmaceutical dosage form provides immediate release of the pharmacologically active agent according to general formula (I) *in vitro* in accordance with Ph. Eur.; and/or
- the pharmaceutical dosage form, wherein the active agent according to general formula (I) is molecularly dispersed; and/or
- the pharmaceutical dosage form contains a self-micro emulsifying formulation; and/or
- the pharmaceutical dosage form comprises a liquid core encapsulated by a solid material, wherein the pharmacologically active agent according to general formula (I) is dispersed in the liquid core; and/or
- the pharmaceutical dosage form, wherein said liquid core further contains an oil; and/or
- the content of said oil is at least 5 wt.-%, based on the total weight of the liquid core; and/or
- the pharmaceutical dosage form contains a coating, preferably a coating that is soluble in gastric juice; and/or
- tₘₐₓ is within the range of from 0.5 to 16 h; and/or
- the ratio AUC₀₋ₜ/ dose is within the range of from 0.5 to 16.5 h/m³; and/or
- ratio Cₘₐₓ / dose is within the range of from 0.06 to 1.69 m⁻³.

A further aspect of the invention relates to the pharmaceutical dosage form according to the invention as described above for use in the treatment of pain.

A further aspect of the invention relates to a method of treating pain comprising the twice daily, once daily, or less frequently, preferably oral administration of the pharmaceutical dosage form according to the invention to a subject in need thereof.

Preferably, the pain is selected from acute, visceral, neuropathic or chronic pain.

### EXAMPLES

The following examples further illustrate the invention but are not to be construed as limiting its scope.

### EXAMPLE 1a:

In order to find a suitable combination of oil, surfactant and hydrophilic solvent, the saturation concentration of the pharmacologically active agent according to formula (I'b) was first determined in a variety of oils as follows:
A saturated solution of said pharmacologically active agent was made by suspending an appropriate amount in 5 g of the respective medium. After removing any trace of undissolved material by filtration through a sintered glass filter (0.45 µm and 0.22 µm), the remaining solution was concentrated under vacuum. The vacuum was maintained until all obvious traces of solvent had been removed, and the flask was then stored over night under high vacuum. The weight of the remaining compound was determined and the saturation concentration of (I'b) in the respective oil was calculated.

The results are displayed in the following table:

**Table 1**

| Oil | Amount of Solvent [g] | Amount of (I'b) (before filtration) [g/5g] | Saturation concentration [mg/g] |
|---|---|---|---|
| Oleic acid | 5 | 0.26061 | 30.0060 |
| Caprylic acid | 5 | 0.13559 | 26.0920 |
| Ethyl oleate | 5 | 0.01373 | 0.8043 |
| Soybean oil | 5 | 0.01587 | 0.8739 |
| Peanut oil | 5 | 0.01400 | 0.9898 |
| Miglyol 812 | 5 | 0.01075 | 0.9400 |
| Captex 355 | 5 | 0.01221 | 0.9615 |
| Labrafac WL 1349 | 5 | 0.01207 | 0.9349 |
| Capryol 90 | 5 | 0.02153 | 3.3547 |
| Capric acid | 5 | 0.12076 | 23.5590 |
| Maisine 35-1 50°C | 5 | 0.04688 | 3.7418 |
| Labrafil M 1944 CS | 5 | 0.04197 | 4.8707 |
| Labrafil M 2125 CS | 5 | 0.04855 | 5.0369 |
| Myristic Acid | 5 | 0.01934 | 3.7878 |

The saturation concentration of compound (I'b) was determined accordingly in a variety of surfactants at 50 °C (filter: 0.45 µm). The results are displayed in the following table:

**Table 2**

| Surfactant | Amount of Solvent [g] | Amount of (I'b) (before filtration) [g/5g] | Saturation concentration [mg/g] |
|---|---|---|---|
| TPGS (PEG-Vit.E succhinate) | 5 | 0.04921 | 8.0491 |
| Gelucire 44/14 | 5 | 0.04265 | 3.7280 |
| Gelucire 50/13 65°C | 5 | 0.05182 | 10.0160 |
| Cremophor EL | 5 | 0.04342 | 9.0519 |
| Cremophor RH40 | 5 | 0.04884 | 9.6462 |
| Tween 20 | 5 | 0.03895 | 7.4428 |
| Tween 60 | 5 | 0.04151 | 8.7866 |
| Tween 80 | 5 | 0.02177 | 4.2641 |
| Labrasol | 5 | 0.05707 | 10.7540 |
| Glycerol monooleate (Peceol) | 5 | 0.01517 | 2.9428 |
| Solutol HS15 | 5 | 0.04921 | 10.1650 |

As suitable oils oleic acid, caprylic acid and capric acid were identified (solubility > 20 mg/g solvent), whereas labrasol, solutol HS 15 and gelucire 50/13 were identified as suitable surfactants (solubility > 10 mg/g solvent).

The saturation concentration of compound (I'b) was determined accordingly in ethanol and selected propylene glycols. The results are displayed in the following table:

**Table 3**

| Solvent | Amount of Solvent [g] | Amount of (I'b) (before filtration) [g/5g] | Saturation concentration [mg/g] |
|---|---|---|---|
| Ethanol | 5 | 0.01498 | 0.1458 |
| Propylenglycol | 5 | 0.01858 | 0.1084 |
| PEG 400 | 5 | 0.03061 | 4.7791 |
| PEG 1500 | 5 | 0.02565 | 8.2633 |
| PEG 4000 | 5 | 0.03992 | 7.6528 |

### EXAMPLE 1b:

According to example 1a, the saturation concentration of compound (I'b) was determined in two-component formulations of one oil (oleic acid, caprylic acid or capric acid) and one surfactant (labrasol, tween 60, solutol HS 15 and gelucire 50/13). Since these two-component formulations are solid at room-temperature, these solubility studies were conducted at 50°C. If these two-component formulations contain sufficient amounts of (I'b), they are also liquid at room temperature. A saturated solution of the two-component formulation is a liquid, as well.

**Table 4**

| Formulation | Ratio surfactant/oil | Amount of (I'b) (before filtration) [g/5g] | Saturation concentration [mg/g] |
|---|---|---|---|
| Labrasol/Caprylic Acid | 5:95 | 0.10308 | 17.24 |
| Labrasol/Caprylic Acid | 10:90 | 0.09126 | 16.87 |
| Labrasol/Caprylic Acid | 20:80 | 0.09185 | 15.89 |
| Labrasol/Caprylic Acid | 40:60 | 0.07167 | 12.3 |
| Labrasol/Oleic Acid | 5:95 | 0.14580 | 28.76 |
| Labrasol/Oleic Acid | 10:90 | 0.12596 | 22.45 |
| Labrasol/Oleic Acid | 20:80 | 0.01154 | 22.85 |
| Labrasol/Oleic Acid | 40:60 | 0.09044 | 19.57 |
| Tween60/Caprylic Acid | 5:95 | 0.09633 | 18.85 |
| Tween60/Caprylic Acid | 10:90 | 0.09832 | 18.98 |
| Tween60/Caprylic Acid | 20:80 | 0.02941 | 7.36 |
| Tween60/Caprylic Acid | 40:60 | 0.03410 | 6.59 |
| Solutol HS15/Caprylic Acid | 5:95 | 0.09329 | 18.32 |
| Solutol HS15/Caprylic Acid | 10:90 | 0.08622 | 16.14 |
| Solutol HS15/Caprylic Acid | 20:80 | 0.07366 | 15.13 |
| Solutol HS15/Caprylic Acid | 40:60 | 0.05841 | 12.39 |
| Solutol HS15/Capryol 90 | 5:95 | 0.05355 | 3.008 |
| Solutol HS15/Capryol 90 | 10:90 | 0.04418 | 3.138 |
| Solutol HS15/Capryol 90 | 20:80 | 0.05118 | 3.650 |
| Solutol HS15/Capryol 90 | 40:60 | 0.04335 | 4.561 |
| Gelucire44/14 / Capryol 90 | 5:95 | 0.04786 | 3.060 |
| Gelucire44/14 Capryol 90 | 10:90 | 0.04515 | 3.312 |
| Geluare44/141 Capryol 90 | 20:80 | 0.04667 | 4.047 |
| Gelucire44/14 / Capryol 90* | 40:60 | 0.06647 | 6.068 |

Accordingly, the saturation concentration of (I'b) in the following three-component formulation was determined:

| | |
|---|---|
| Solutol® HS 15 | 60% (w/w) |
| Miglyol 812 | 20% (w/w) |
| Ethanol (abs.) | 20% (w/w) |

The saturation concentration of (I'b) in said formulation was determined to be 1.3 mg/g.

### EXAMPLE 2:

The phase behavior of the oil/surfactant mixtures according to example 1 b was determined in presence of an aqueous medium.

For that purpose, 20 g of the respective two-component mixture was made by mixing the respective amounts of oil and surfactant. 0.5 g of the mixture was then added to 250 g of water or 250 g of gastric juice, respectively.

The formation of an emulsion in the aqueous medium was observed, described visually (qualitative extent of turbidity) and measured quantitatively by means of a conventional turbidity photometer.

**Table 5**

| | ratio surfactant/oil [wt.%/ wt.-%] | Appearance of the formulation (dear, turbid, colour,...) | | | | |
|---|---|---|---|---|---|---|
| | | in absence of aqueous media | in 250 mL H₂O | | in 250 mL gastric juice | |
| | | | [TE/F] | | [TE/F] | |
| Labrasol/ Caprylic Add | | dear | 25 | emulsion | 32 | oil droplets, turbid |
| Labrasol/ Caprylic Acid | 10:90 | dear | 23 | emulsion | 2.9 | oil droplets, clear |
| Labrasol/ Caprylic Acid | 20:80 | dear | 21 | emulsion | 11 | oil droplets, dear |
| Labrasol/ Caprylic Acid | 40:60 | dear | 82 | emulsion | 15 | oil droplets, dear |
| Labrasol/ Oleic Add | 5:95 | dear | 36 | emulsion, clear | 12.2 | oil droplets, turbid |
| Labrasol/ Oleic Acid | 10:90 | dear | 46 | emulsion, dear | 19 | oil droplets, turbid |
| Labrasol/ Oleic Acid | 20:80 | dear | 41 | emulsion, dear | 33 | oil droplets, turbid |
| Labrasol/ Oleic Acid | 40:60 | clear | 124 | emulsion, dear | 71 | oil droplets, turbid |
| Labrasol/ Capric Acid | 5:95 | solid, white, crystalline | 28 | n.d. | 9 | Solid, partly fibrous matter |
| Labrasol/ Capric Acid | 10:90 | solid, white, crystalline | 34 | n.d. | 7.7 | Solid, partly fibrous matter |
| Labrasol/ Capric Acid | 20:80 | solid, white, crystalline | 27 | n.d. | 10.3 | Solid, partly fibrous matter |
| Labrasol/ Capric Acid | 40:60 | solid, white, clear | 60 | n.d. | 11 | Solid, partly fibrous matter |
| Tween60/ Caprylic Acid | 5:95 | dear, slightly yellowish | 33 | turbid, drop formation | 19 | small droplets, turbid |
| Tween60/ Caprylic Acid | 10:90 | dear, slightly yellowish | 34 | turbid, drop formation | 36 | droplets, turbid |
| Tween60/ Caprylic Acid | 20:80 | dear, yellowish | 69 | opaque | 80 | flocculate |
| Tween60/ Caprylic Acid | 40:60 | clear, deep yellow | 162 | turbid, no drop formation visible | 155 | Turbid, no drop formation visible |
| Tween60/ Oleic Acid | 5:95 | clear, slightly yellowish | 404 | milky, upper layer, flocculate | 350 | flocculate |
| Tween60/ Oleic Acid | 10:90 | dear, slightly yellowish | >680 | milky, upper layer, flocculate | 776 | flocculate |
| Tween60/ Oleic Acid | 20:80 | dear, slightly yellowish | 614 | milky, upper layer, flocculate | 876 | flocculate |
| Tween60/ Oleic Acid | 40:60 | clear, slightly yellowish | 910 | milky, upper layer, flocculate | 520 | flocculate |
| Tween60/ Capric Acid | 5:95 | solid, yellowish-white, crystalline | 34 | solid, fibrous | 17 | solid upper layer, flocculate |
| Tween60/ Capric Acid | 10:90 | Solid, yellowish-white, crystalline | 35 | containing solid matter | 25 | solid upper layer, flocculate |
| Tween60/ Capric Acid | 20:80 | solid, yellowish-white, crystalline | 72 | deeply turbid | 85 | solid upper layer, flocculate |
| Tween60/ Capric Acid | 40:60 | Solid, yellowish-white | 646 | turbid, no drop formation visible | 279 | solid upper layer, flocculate |
| Solutol HS15/ Caprylic Acid | 5:95 | dear | 27 | floating oily drops | 8.1 | oil droplets |
| Solutol HS15/ Caprylic Acid | 10:90 | dear | 12,4 | floating oily drops | 11 | oil droplets |
| Solutol HS15/ Caprylic Acid | 20:80 | dear | 28 | emulsoid, slightly turbid | 18 | emulsoid, small droplets |
| Solutol HS15/ Caprylic Acid | 40:60 | dear | 54 | opaque, (microemulsion) | 98 | opaque (microemulsion) |
| Solutol HS15/ Oleic Acid | 5:95 | dear | 55 | drop formation, opaque | 30 | large droplets |
| Solutol HS15/ Oleic Acid | 10:90 | clear | 36 | flocculate, turbid | 60 | flocculate |
| Solutol HS15/ Oleic Acid | 20:80 | dear | 704 | flocculate, turbid | 450 | flocculate |
| Solutol HS15/ Oleic Acid | 40:60 | 2 phases: dear, turbid | 1267 | flocculate, turbid | 777 | flocculate, deeply turbid |
| Solutol HS15/ Capric Acid | 5:95 | solid, white, clear | 8,5 | needles | 11.4 | solid upper layer |
| Solutol HS15/ Capric Acid | 10:90 | solid, white, clear | 21 | needles | 8.8 | solid upper layer |
| Solutol HS15/ Capric Acid | 20:80 | solid, white, clear | 23 | needles | 13.7 | solid upper layer |
| Solutol HS15/ Capric Acid | 40:60 | 2 phases: liquid, dear; crystalline, white | 144 | turbid, upper layer | 93 | small crystal needles |
| Gelucire 50/13/ Caprylic Acid | 5:95 | Clear | 26 | transparent, small drops | 26 | emulsoid, turbid |
| Gelucire 50/13/ Caprylic Acid | 10:90 | Clear; crystalline, white, precipitate | 12,1 | transparent, small drops | 24 | emulsoid, turbid |
| Gelucire 50/13/ Caprylic Acid | 20:80 | Clear; crystalline, white, precipitate | 19 | transparent, drops of variable size | 65 | emulsoid, turbid |
| Gelucire 50/13/ Caprylic Acid | 40:60 | Clear; crystalline, white, precipitate | 90 | turbid, flocculate | 87 | opaque, particles |
| Gelucire 50/13/ Oleic Acid | 5:95 | Clear; crystalline, white, precipitate | 335 | upper layer, white foam | 450 | emulgator seems not to |
| Gelucire 50/13/ Oleic Acid | 10:90 | Clear; crystalline, white, precipitate | 465 | upper layer, white foam | 1031 | be soluble emulgator seems not to be soluble |
| Gelucire 50/13/ Oleic Acid | 20:80 | Clear; crystalline, white, precipitate, highly viscous | 1050 | upper layer, white foam | 260 | emulgator seems not to be soluble |
| Gelucire 50/13/ Oleic Acid | 40:60 | Clear; crystalline, white precipitate, highly viscous | 403 | upper layer, white foam | 263 | emulgator seems not to be soluble |
| Gelucire 50/13/ Capric Acid | 5:95 | solid, white, crystalline | 7,3 | Solid upper layer | 32 | n.d. |
| Gelucire 50/13/ Capric Acid | 10:90 | solid, white, crystalline | 21 | Solid upper layer | 57 | n.d. |
| Gelucire 50/13/ Capric Acid | 20:80 | solid, white, crystalline | 62 | Solid upper layer | 58 | n.d. |
| Gelucire 50/13/ Capric Acid | 40:60 | solid, white, crystalline | 520 | Solid upper layer | 433 | n.d. |

The extent of turbidity is an indication for the size of the droplets of an emulsion. Micro- or nanoemulsions appear clear to the naked eye and do not show turbidity, since the size of the droplets is too small for refracting visible light.

As a result, two-component mixtures of labrasol and caprylic acid, labrasol and oleic acid, tween 60 and caprylic acid, as well as solutol HS 15 and caprylic acid formed clear solutions in absence of any aqueous media. All of them formed emulsions in water and gastric juice. Tween 60/ caprylic acid (40:60) and solutol HS 15/ caprylic acid (40:60) showed the best phase behavior in aqueous media forming clear microemulsions whithout visible drop formation in water and in gastric juice.

Emulsions which gave promising results in the previous study were then subjected to particle size analysis (using a laser-based particle size analyzer type Zetasizer NanoZS, Malvern Instruments) with a particle size measurement range of 0.02 to 2,000 µm. Particle size was calculated from the volume size distribution.

**Tables 6-14**

| Labrasol-oleic acid 40:60 in 250g H₂O | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 326.2 | 3.1 | 100.2 | 96.9 | 0 | 0 |
| 2 | 169.8 | 100 | 0 | 0 | 0 | 0 |
| 3 | 2956 | 0.1 | 152 | 99.9 | 0 | 0 |

| Labrasol-oleic acid 40:60 in 250g gastric juice | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 242.6 | 100 | 0 | 0 | 0 | 0 |
| 2 | 243.8 | 100 | 0 | 0 | 0 | 0 |
| 3 | 265.8 | 100 | 0 | 0 | 0 | 0 |
| | | | | | | |

| Labrasol-oleic acid 5:95 in 250g H₂O | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 0 | 0 | 0 | 0 | 0 | 0 |
| 2 | 6.411 | 100 | 0 | 0 | 0 | 0 |
| 3 | 19.35 | 100 | 0 | 0 | 0 | 0 |
| | | | | | | |

| Solutol- caprylic acid 40:60 in 250g H₂O | | | | | | |
|---|---|---|---|---|---|---|
| Measureme nt | Peak 1 diameter [nm] | Peak 1 occurence [%] | Peak 2 diameter [nm] | Peak 2 occurence[%] | Peak 3 diameter [nm] | Peak 3 occurence [%] |
| 1 | 289.7 | 8.4 | 2938 | 0 | 94.4 | 91.6 |
| 2 | 154.9 | 96 | 772.2 | 3.2 | 0 | 0 |
| 3 | 175.7 | 97.6 | 982.8 | 2.4 | 0 | 0 |
| | | | | | | |

| Solutol- caprylic acid 40:60 in 250g gastric juice | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 142 | 100 | 0 | 0 | 0 | 0 |
| 2 | 107.8 | 100 | 0 | 0 | 0 | 0 |
| 3 | 206 | 100 | 0 | 0 | 0 | 0 |
| | | | | | | |

| Tween 60- caprylic acid 40:60 in 250g H₂O | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 62.06 | 100 | 0 | 0 | 0 | 0 |
| 2 | 79.3 | 100 | 0 | 0 | 0 | 0 |
| 3 | 53.32 | 100 | 0 | 0 | 0 | 0 |
| | | | | | | |

| Tween 60- caprylic acid 40:60 in 250g gastric juice | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 52.24 | 99.6 | 204.5 | 0.4 | 0 | 0 |
| 2 | 49.07 | 99.2 | 146.6 | 0.8 | 0 | 0 |
| 3 | 46.15 | 98.4 | 117.3 | 1.6 | 546.6 | 0 |
| | | | | | | |

| Tween 60- caprylic acid 20:80 in 250g H₂O | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 151.3 | 0.1 | 28.37 | 99.9 | 0 | 0 |
| 2 | 198.2 | 100 | 0 | 0 | 0 | 0 |
| 3 | 186.3 | 100 | 0 | 0 | 0 | 0 |

| Tween 60- caprylic acid 20:80 in 250g gastric juice | | | | | | |
|---|---|---|---|---|---|---|
| Measureme nt | Peak 1 diameter [nm] | Peak 1 occurence [%] | Peak 2 diameter [nm] | Peak 2 occurence [%] | Peak 3 diameter [nm] | Peak 3 occurence [%] |
| 1 | 1218 | 0.2 | 59.09 | 99.8 | 0 | 0 |
| 2 | 442.2 | 100 | 0 | 0 | 0 | 0 |
| 3 | 892.6 | 0.3 | 65.8 | 99.7 | 0 | 0 |

### EXAMPLE 3:

The stability of compound (I'b) in a variety of oils and surfactants was determined under stress (elevated temperature):
For this study, 50 mg of said compound was dissolved in 50 g of the respective medium and objected to elevated temperatures for 6 weeks. The same study was conducted using 500 mg of compound (I'b). After this period, the corresponding demethylated derivative (I'a) and/or the corresponding cis/trans-isomerized derivatives were identified as main degradation products.

The results show that the lowest degradation occurred in Solutol, Capryol and Gelucire. In general, the degradation was lower in the higher dosed samples.

**Table 15**

| | 50 mg in 50g Oleic acid | 50 mg in 50g Caprylic acid | 50 mg in 50g Capric acid | 50 mg in 50g Gelucire | 41 mg in 41g Tween 60 | 50 mg in 50g Labrasol | 50 mg in 50g Solutol | 50 mg in 50g Capryol |
|---|---|---|---|---|---|---|---|---|
| Content of (I'b) | | | | | | | | |
| in the assay | 170.01% | 100.39% | 186.13% | 100.46% | 93.05% | 99.55% | 100.11% | 99.70% |
| after 2 weeks at 25°C | 175.72% | 101.01% | 101.68% | 101.29% | 96.73% | 99.94% | 102.72% | 101.06% |
| 2 weeks at 40°CF | 176.43% | 100.68% | 101.10% | 101.09% | 92.43% | 95.69% | 102.13% | 100.67% |
| 4 weeks at 25°C | 156.64% | 101.43% | 97.62% | 102.62% | 92.22% | 98.33% | 100.25% | 99.46% |
| 4 weeks at 40°CF | 143.56% | 101.11% | 91.46% | 102.56% | 89.09% | 91.93% | 100.08% | 99.07% |
| 6 weeks at 25°C | 156.85% | 101.69% | 120.87% | 100.75% | 91.45% | 97.63% | 100.45% | 99.86% |
| 6 weeks at 40°CF | 141.66% | 100.93% | 94.19% | 100.41% | 86.93% | 89.87% | 99.69% | 99.36% |

| Purity | | | | | | | | |
|---|---|---|---|---|---|---|---|---|
| in the assay | 93.85% | 95.26% | 94.93% | 99.34% | 96.63% | 98.36% | 98.83% | 97.44% |
| compound (I'a) | 4.93% | - | - | 0.66% | - | 1.64% | - | 0.22% |
| cis isomer of (I'a) | - | - | - | - | - | - | - | - |
| Amount other | 1.23% | 4.74% | 5.07% | - | 3.37% | - | 1.08% | 2.34% |
| cis isomer of (I'b) | - | - | - | - | - | - | 0.09% | - |
| after 2 weeks at 25°C | 93.22% | 95.19% | 94.71% | 99.60% | 97.01% | 98.22% | 98.92% | 97.51% |
| compound (I'a) | 5.75% | - | - | - | - | 1.71% | - | 0.13% |
| cis isomer of (I'a) | - | - | - | - | - | - | - | - |
| amount other | 1.03% | 4.81% | 3.95% | 0.40% | 2.99% | 0.06% | 1.01% | 2.36% |
| cis isomer of (I'b) | - | - | 1.35% | - | - | - | 0.07% | - |
| after 2 weeks at 40°CF | 88.45% | 95.12% | 94.57% | 99.30% | 94.62% | 95.11% | 98.81% | 97.37% |
| compound (I'a) | 9.69% | - | - | - | - | 4.29% | - | 0.22% |
| cis isomer of (I'a) | - | - | - | - | - | - | - | - |
| amount other | 1.77% | 4.88% | 4.13% | 0.70% | 5.38% | 0.61% | 1.03% | 2.41% |
| cis isomer of (I'b) | 0.10% | - | 1.30% | - | - | - | 0.17% | - |
| after 4 weeks at 25°C | 89.17% | 94.51% | 98.96% | 99.36% | 96.96% | 97.07% | 99.12% | 99.29% |
| compound (I'a) | 10.72% | 0.05% | - | 0.59% | - | 2.82% | 0.04% | 0.20% |
| cis isomer of (I'a) | - | - | 1.04% | 0.03% | - | 0.03% | - | - |
| amount other | 0.12% | 5.45% | - | 0.03% | 3.03% | 0.08% | 0.84% | 0.51% |
| cis isomer of (I'b) | - | - | - | - | - | - | - | - |
| after 4 weeks at 40°CF | 84.27% | 97.30% | 99.13% | 98.96% | 94.12% | 92.02% | 98.90% | 98.10% |
| compound (I'a) | 15.48% | 0.08% | - | 0.98% | - | 7.87% | 0.16% | 0.34% |
| cis isomer of (I'a) | - | - | - | 0.03% | - | 0.02% | - | |
| amount other | 0.25% | 2.62% | 0.87% | 0.03% | 5.87% | 0.09% | 0.95% | 1.57% |
| cis isomer of (I'b) | - | - | - | - | - | - | - | - |
| after 6 weeks at 25°C | 88.41% | 96.57% | 93.36% | 99.44% | 96.89% | 96.71% | 99.15% | 98.54% |
| compound (I'a) | 11.52% | 0.32% | 5.63% | 0.53% | 2.78% | 3.24% | 0.08% | 0.16% |
| cis isomer of (I'a) | - | - | 0.95% | 0.03% | - | - | - | - |
| amount other | 0.07% | 3.11% | 0.06% | - | 0.33% | 0.04% | 0.77% | 1.29% |
| cis isomer of (I'b) | - | - | - | - | - | - | - | - |
| after 6 weeks at 40°CF | 84.99% | 96.06% | 92.74% | 98.81% | 93.60% | 90.17% | 98.82% | 98.22% |
| compound (I'a) | 14.45% | 0.5% | 6.34% | 1.17% | 5.99% | 9.78% | 0.20% | 0.37% |
| cis isomer of (I'a) | - | - | 0.92% | 0.02% | - | - | - | - |
| amount other | 0.57% | 3.43% | - | - | 0.41% | 0.05% | 0.98% | 1.42% |
| cis isomer of (I'b) | - | - | - | - | - | - | - | - |
| | | | | | | | | |

| | 500 mg in 50g Oleic acid | 500 mg in 50g Caprylic acid | 500 mg in 50g Capric acid | 400 mg in 50g Gelucire | 400 mg in 50g Labrasol | 400 mg in 50g Solutol | 350 mg in 50g Tween 60 | 100 mg in 50g Capryol |
|---|---|---|---|---|---|---|---|---|
| Content of (I'b) | | | | | | | | |
| in the assay | 102.13% | 100.13% | 101.20% | 100.93% | 98.55% | 98.18% | 99.41% | 99.46% |
| after 2 weeks at 25°C | 102.61% | 100.12% | 102.50% | 102.67% | 97.36% | 100.21% | 94.96% | 100.97% |
| 2 weeks at 40°CF | 100.48% | 100.22% | 102.19% | 101.53% | 97.09% | 89.65% | 105.18% | 101.57% |
| 4 weeks at 25°C | 96.79% | 99.46% | 98.89% | 100.90% | 97.63% | 98.41% | 99.26% | 100.10% |
| 4 weeks at 40°CF | 95.70% | 99.62% | 100.76% | 100.54% | 95.60% | 80.83% | 89.08% | 99.63% |
| 6 weeks at 25°C | 93.24% | 99.77% | 112.08% | 99.77% | 97.41% | 99.16% | 78.26% | 99.62% |
| 6 weeks at 40°CF | 94.39% | 99.02% | 100.26% | 99.16% | 96.52% | 88.83% | 84.54% | 99.22% |

| Purity | | | | | | | | |
|---|---|---|---|---|---|---|---|---|
| in the assay | 94.62% | 99.35% | 98.94% | 99.91% | 98.78% | 99.82% | 99.02% | 98.00% |
| compound (I'a) | 4.62% | - | - | 0.09% | 1.22% | - | 0.81% | 0.29% |
| cis isomer of (I'a) | - | - | - | - | - | - | - | - |
| Amount other | 0.76% | 0.65% | 1.06% | - | - | 0.11% | - | 1.71% |
| cis isomer of (I'b) | - | - | - | - | - | 0.07% | 0.17% | - |
| after 2 weeks at 25°C | 94.40% | 99.61% | 100.00% | 99.91% | 98.77% | 99.81% | 98.98% | 98.79% |
| compound (I'a) | 4.96% | - | - | 0.09% | 1.23% | - | 0.73% | 0.13% |
| cis isomer of (I'a) | - | - | - | - | - | - | - | - |
| amount other | 0.64% | 0.39% | - | - | - | 0.12% | 0.30% | 1.08% |
| cis isomer of (I'b) | - | - | - | - | - | 0.06% | - | - |
| after 2 weeks at 40°CF | 91.26% | 99.61% | 99.92% | 99.90% | 97.75% | 99.74% | 98.09% | 98.67% |
| compound (I'a) | 7.09% | - | - | 0.10% | 2.25% | 0.06% | 1.26% | 0.20% |
| cis isomer of (I'a) | - | - | - | - | - | - | - | - |
| amount other | 1.57% | 0.39% | - | - | - | 0.09% | 0.65% | 1.13% |
| cis isomer of (I'b) | 0.08% | - | 0.08% | - | - | 0.11% | - | - |
| after 4 weeks at 25°C | 93.55% | 99.75% | 99.91% | 99.86% | 98.10% | 99.78% | 98,82% | 98.99% |
| compound (I'a) | 6.33% | - | - | 0.11% | 1.87% | 0.05% | 0.04% | 0.15% |
| cis isomer of (I'a) | - | - | - | - | - | 0.06% | - | - |
| amount other | 0.12% | 0.25% | 0.09% | 0.03% | 0.03% | 0.12% | 0.84% | 0.85% |
| cis isomer of (I'b) | - | - | - | - | - | - | - | - |
| after 4 weeks at 40°CF | 91.70% | 99.71% | 99.90% | 99.79% | 96.79% | 99.64% | 97.99% | 98.79% |
| compound (I'a) | 8.30% | 0.03% | 0.02% | 0.17% | 3.17% | 0.10% | 1.76% | 0.29% |
| cis isomer of (I'a) | - | - | - | - | - | 0.15% | 0.25% | |
| amount other | 0.10% | 0.25% | 0.08% | 0.03% | 0.04% | 0.11% | - | 0.93% |
| cis isomer of (I'b) | - | - | - | - | - | - | - | - |
| after 6 weeks at 25°C | 93.26% | 99.72% | 99.85% | 99.89% | 97.92% | 99.71% | 98.79% | 99.05% |
| compound (I'a) | 6.65% | 0.02% | 0.05% | 0.08% | 2.06% | 0.11% | 1.08% | 0.16% |
| cis isomer of (I'a) | - | 0.21% | 0.05% | 0.03% | 0.02% | 0.13% | 0.13% | - |
| amount other | 0.10% | 0.05% | 0.04% | - | - | 0.06% | - | 0.79% |
| cis isomer of (I'b) | - | - | - | - | - | - | - | - |
| after 6 weeks at 40°CF | 92.03% | 99.70% | 99.01% | 99.82% | 96.52% | 99.80% | 97.99% | 98.91% |
| compound (I'a) | 7.88% | 0.04% | 0.31% | 0.16% | 3.46% | 9.78% | 1.85% | 0.27% |
| cis isomer of (I'a) | - | 0.21% | 0.61% | 0.02% | 0.02% | - | 0.16% | - |
| amount other | 0.09% | 0.04% | 0.08% | - | - | 0.05% | - | 0.83% |
| cis isomer of (I'b) | - | - | - | - | - | - | - | - |

### EXAMPLE 4:

The ability and performance of Solutol HS 15/Capryol mixtures and Gelucire 44/14/Capryol mixtures to (micro)emulsify, each containing compound (I'b), was determined next.

For that purpose, 20 g of the respective formulation was made by mixing the respective amounts of oil, surfactant and compound (I'b). 0.5 g of the resulting formulation were then exposed to 250 mL of water or 250 mL of gastric juice and the resulting emulsion formation was measured qualitatively (visually) and quantitatively by means of a conventional turbidity photometer.

**Table 16**

| | | | Appearance of the formulation (clear, turbid, colour,...) | | | | |
|---|---|---|---|---|---|---|---|
| | ratio surfactant/oil | Solubility of (I'b) (in surfactant/oil) | in absence of aqueous media | in 250 mL H₂O | | | in 250 mL gastric juice |
| | [wt.-%/wt.-%] | [mg/g] | | [TE/F] | | [TE/F] | |
| Solutol HS/ Capryol | 5:95 | 3.01 | clear | 169 | emulsion, transparent, slightly turbid | 313 | milky, opaque, uniformly turbid |
| Solutol HS/ Capryol | 10:90 | 3.14 | clear | 430 | emulsion, more turbid than previous sample | 411 | milky, opaque |
| Solutol HS/ Capryol | 20:80 | 3.65 | clear | 180 | turbid, floating particles, vanish during shaking | 238 | transparent, slightly opaque, microemulsion |
| Solutol HS/ Capryol | 40: 60 | 4.56 | clear | 66 | turbid, floating particles, vanish during shaking | 56 | transparent, microemulsion, more turbid than previous sample |
| Gelucire 44/14/ Capryol | 5:95 | 3.06 | clear | 101 | clear, emulsion | 136 | Turbid with particles, rather suspension |
| Gelucire 44/14/ Capryol | 10:90 | 3.31 | transparent with streaks | 85 | slightly opaque, emulsion | 290 | Turbid with particles, rather suspension |
| Gelucire 44/14/ Capryol | 20:80 | 4.05 | white, solid, flocalate, turbid | 136 | opaque, emulsoid | 338 | Turbid with particles, rather suspension |
| Gelucire 44/14/ Capryol | 40: 60 | 6.07 | Solid and liquid parts at 25°C, saturated solution of (I'b) is liquid | 31 | milky, emulsoid | 50 | Turbid with particles, rather suspension |

The formation of a microemulsion was observed in those samples which contained Solutol HS 15 and Capryol in ratios of 20:80 and 40:60, respectively.

### EXAMPLE 5:

A formulation was formulated from the following composition:

| | |
|---|---|
| Solutol® HS 15 | 60% (w/w) |
| Miglyol 812 | 20% (w/w) |
| Ethanol (abs.) | 20% (w/w) |

By adding the appropriate amount of compound (I'a) to the said formulation, solutions of three different concentrations were prepared:

**Table 17**

| | C-1 (placebo) | I-1 | I-2 | I-3 |
|---|---|---|---|---|
| Formulation | 1 mL | 1 mL | 1 mL | 1 mL |
| Compound (1'a) | - | 0.1 mg | 1.0 mg | 10 mg |
| Concentration of (1'a) in the formulation | | 0.01 wt.-% | 0.1 wt.-% | 1.0 wt.-% |

1 mL of each of these mixtures was diluted with 20 mL of water. Additional water was sequentially added to the respective solution in 5 mL portions and the turbidity process was analyzed by means of a conventional turbidity spectrometer:

**Table 18**

| | | [TE/F] | | |
|---|---|---|---|---|
| | C-1 (placebo) | I-1 | I-2 | I-3 |
| +0 mL H₂O | 58 | 54 | 57 | 55 |
| +5 mL H₂O | 51 | 47 | 51 | 47 |
| +10 mL H₂O | 44 | 42 | 49 | 41 |
| +15 mL H₂O | 40 | 36 | 42 | 37 |
| +20 mL H₂O | 36 | 29 | 36 | 32 |
| +25 mL H₂O | 32 | 27 | 32 | 29 |
| +30 mL H₂O | 30 | 25 | 28 | 26 |
| +35 mL H₂O | 28 | 23 | 27 | 24 |
| +40 mL H₂O | 25 | 21 | 27 | 24 |
| +45 mL H₂O | 24 | 20 | 22 | 21 |
| +50 mL H₂O | 22 | 19 | 21 | 19 |
| +55 mL H₂O | 21 | 17.7 | 20 | 18 |
| +60 mL H₂O | 20 | 16.7 | 20 | 17.3 |
| +65 mL H₂O | 19 | 16.2 | 20 | 16.4 |
| +70 mL H₂O | 18.1 | 15.4 | 16.9 | 15.5 |
| +75 mL H₂O | 17.5 | 14.5 | 15.8 | 15.2 |
| +80 mL H₂O | 16.2 | 14.2 | 15.4 | 14 |
| +85 mL H₂O | 15.9 | 13.4 | 16 | 14 |
| +90 mL H₂O | 15.4 | 13.7 | 16 | 13.5 |
| + 95 mL H₂O | 14.8 | 13.1 | 14.1 | 12.2 |
| +100 mL H₂O | 14.3 | 12.3 | 13.5 | 11.9 |
| +105 mL H₂O | 13.6 | 12.5 | 14.4 | 11.1 |
| +110 mL H₂O | 13.2 | 11.6 | 12.6 | 11.4 |
| +115 mL H₂O | 12.9 | 11.8 | 12.6 | 10.2 |
| +120 mL H₂O | 12.5 | 11.2 | 12.1 | 10.2 |
| +125 mL H₂O | 12 | 10.8 | 15.5 | 9.7 |
| +130 mL H₂O | 11.9 | 10.4 | 12.7 | 9.3 |
| +135 mL H₂O | 11.6 | 10.5 | 11.3 | 9.3 |
| +140 mL H₂O | 11.5 | 9.6 | 10.8 | 9.2 |
| +145 mL H₂O | 11.1 | 10.4 | 10.7 | 8.7 |
| +150 mL H₂O | 10.8 | 10 | 13 | 9 |
| +155 mL H₂O | 10.7 | 9.2 | 10.1 | 8.3 |
| +160 mL H₂O | 10.3 | 9.2 | 9.8 | 8.2 |
| +165 mL H₂O | 10 | 10 | 9.3 | 8.1 |
| +170 mL H₂O | 9.8 | 8.6 | 9.3 | 7.9 |
| +175 mL H₂O | 9.5 | 8.6 | 9.2 | 7.7 |
| +180 mL H₂O | 9.2 | 8.2 | 10.5 | 7.8 |
| +185 mL H₂O | 9 | 8 | 8.5 | 8.2 |
| +190 mL H₂O | 9.4 | 8.3 | 8.4 | 7 |
| +195 mL H₂O | 9.4 | 7.6 | 8.2 | 7 |
| +200 mL H₂O | 9 | 7.8 | 8.5 | 6.9 |
| +205 mL H₂O | 8.4 | 7.8 | 7.8 | 6.5 |
| +210 mL H₂O | 8.4 | 7.3 | 7.7 | 6.5 |
| +215 mL H₂O | 8.1 | 7 | 7.5 | 6.3 |
| +220 mL H₂O | 8.8 | 7 | 7.8 | 6 |
| +225 mL H₂O | 8.3 | 6.9 | 7.6 | 6 |
| +230 mL H₂O | 8.1 | 6.7 | 7.2 | 5.9 |

The experimental findings show that the turbidity profile of formulation according to the comparative sample C-1 is comparable to the turbidity profiles of the formulations according to the inventive examples I-1, I-2 and I-3, meaning that the presence of compound (I'a) hardly has an influence on the ability to (micro)emulsify of these formulations

### EXAMPLE 6:

The following SMEDDS (self-microemulsifying drug delivery system) formulation was prepared:

| | |
|---|---|
| Solutol® HS 15 | 60% (w/w) |
| Miglyol 812 | 20% (w/w) |
| Ethanol (abs.) | 20% (w/w) |

From the SMEDDS formulation and appropriate amounts of compound (1'b), solutions were prepared, that were then put into hard gelatin capsules. According to the following composition capsules of two dosages were prepared (50 µg and 400 µg):

**Table 19**

| Ingredient | % (m/m), Dosage 50µg | Amount per capsule [mg] | % (m/m), Dosage 400µg | Amount per capsule [mg] |
|---|---|---|---|---|
| Formulation SMEDDS | 99.986 | 349.95 | 99.886 | 349.6 |
| Compound (I'b) | 0.014 | 0.05 | 0.114 | 0.4 |
| Total | 100.0 | 350.0 | 100.0 | 350.0 |

Two batches of about 1000 of these hard gelatin capsules were manufactured for each of both dosages and packaged in brown glasses with screw caps. The SMEDDS formulations were put into empty hard gelatin capsules (size 0) and subjected to stability testing studies at 25°C/60% relative humidity (RH) (long-term storage conditions) and 40°C/75% RH (accelerated storage conditions). Samples were charged in stability chambers with humidity and temperature control. They were withdrawn at specified intervals for analysis over a period of 6 months. Drug content of the capsules was analyzed using a previously developed and validated stability-indicating HPLC method. Besides the chemical stability of the drug, the dissolution profile of the dosage form was also studied.

The testing results revealed that all measured parameters (unity of the drug content, the drug's purity, release of the drug) met the demands of the ICH and FDA guidelines. The determined content of the decomposition product 6'-fluoro-4-phenyl-4',9'-dihydro-3'H-spiro[cyclohex-3-ene-1,1'-pyrano[3,4-b]indole] after storage at 40°C and 75% rh was for dosage 50 µg 0.16% after 1 month, 0.25% after 3 months and 0.42% after 6 months; and for dosage 400 µg 0.09% after 1 month, 0.12% after 3 months and 0.25% after 6 months.

### EXAMPLE 7 (not according to the invention):

Clinical studies were conducted to determine the analgesic efficacy and tolerability of single doses of the compound according to formula (I'b) (200 µg, 400 µg and 600 µg, based on the content of the free base; hemicitrate oral solution of compound (I'b) in Macrogol 400) compared to that of morphine (60 mg, controlled-released form) and placebo in patients with acute post-operative pain following orthopedic surgery (bunionectomy).

For this purpose, 258 patients of either sex were included in a randomized, placebo-controlled, double-blind clinical trial in parallel groups. Treatment groups were well-balanced with respect to demographics and baseline characteristics with a slight imbalance in baseline pain and ethnicity.

After surgery, all patients were initially treated with local post-operative anesthesia via a popliteal block. Due to different kinetics of the compound according to formula (I'b) and morphine, the patients were then treated with either one of the two drugs or with placebo at slightly different times:
One hour before the popliteal block was stopped, patients were randomized and part of them were dosed with a single dose of the compound according to formula (I'b) (200 µg, 400 µg or 600 µg) or placebo, while the others received morphine or placebo 2 hours after the popliteal block had been stopped.

The primary efficacy assessment endpoint was the absolute pain intensity over a 24 hour period. Pain intensity was measured using an 11-point numerical rating scale (NRS). At each time point, patients were instructed to evaluate their current pain intensity relative to an 11-point numerical rating scale. A score of zero represented no pain and a score of 10 represented worst possible pain. Missing scheduled pain assessments for the patients were imputed with the last observation carried forward (LOCF). The resulting averaged NRS values over the 24 hour period are depicted in Figure 1.

Sum of pain intensity differences over different time periods were analyzed using an analysis of covariance (ANCOVA) model with factors for treatment and site and baseline pain intensity score (using the pain intensity NPRS score). Only subjects with non-missing baseline pain intensity were included. A summary of the analysis for the 2 to 10 hour period is presented in Table 20. The resulting p-values are summarized in Table 21.

**Table 20**

| | n | LS mean | SE | LS mean Δplacebo | SE | P-value |
|---|---|---|---|---|---|---|
| placebo | 45 | 49.13 | 2.85 | | | |
| compound (I'b) 200 µg | 52 | 46.05 | 2.78 | -3.08 | 3.49 | 0.3776 |
| compound (I'b) 400 µg | 47 | 35.28 | 2.81 | -13.85 | 3.57 | 0.0001 |
| compound (I'b) 600 µg | 55 | 35.15 | 2.67 | -13.98 | 3.45 | < 0.0001 |
| morphine, controlled-release 60 mg | 49 | 42.01 | 2.83 | -7.12 | 3.54 | 0.0454 |

| | | | | | | |
|---|---|---|---|---|---|---|
| LS mean: least squares means; SE: statistical error | | | | | | |

**Table 21**

| p-values (sum of pain intensity differences) | 2-6 h | 2-10 h | 2-12 h | 2-14 h | 2-18 h | 2-24 h |
|---|---|---|---|---|---|---|
| compound (I'b) 200 µg | 0.4514 | 0.3776 | 0.3387 | 0.3427 | 0.3205 | 0.2923 |
| compound (I'b) 400 µg | 0.0009 | 0.0001 | < 0.0001 | 0.0001 | 0.0005 | 0.0008 |
| compound (I'b) 600 µg | 0.0009 | <0.0001 | < 0.0001 | <0.0001 | <0.0001 | 0.0001 |
| morphine, controlled-release 60 mg | 0.4664 | 0.0454 | 0.0084 | 0.0036 | 0.0014 | 0.0005 |

Accordingly, on the primary parameter, a statistically significant difference was observed between groups that had received a 400 µg or 600 µg dose of compound (I'b) and placebo groups, whereas no statistically significant difference was observed for groups that had received a 200 µg dose of compound (I'b).

Tables 22 and 23 summarize the treatment emergent adverse events (TEAE(s)) experienced by the five treatment groups.

**Table 22**

| | Placebo | compound (I'b) 200 µg | compound (I'b) 400 µg | compound (I'b) 600 µg | morphine 60 |
|---|---|---|---|---|---|
| subjects with TEAE(s) (n (%)) | 32 (68.1) | 37 (67.3) | 38 (77.6) | 48 (84.2) | 46 (92.0) 46 (92.0) |
| related (n (%)) | 17 (36.2) | 24 (43.6) | 32 (65.3) | 43 (75.4) | 42 (84.0) |
| serious (n (%)) | 1 (2.1) | 0 | 0 | 0 | 0 |
| total number of TEAE's (n) | 74 | 75 | 125 | 198 | 144 |
| related (n (%)) | 32 (43.2) | 37 (49.3) | 74 (59.2) | 146 (73.7) | 99 (68.8) |
| subjects with SAE's | 1 (2.1) | 0 | 0 | 0 | 0 |
| deaths | 0 | 0 | 0 | 0 | 0 |

| | | | | | |
|---|---|---|---|---|---|
| TEAE: treatment emergent adverse event; SAE: serious adverse event | | | | | |

**Table 23**

| | Placebo | compound (I'b) 200 µg | compound (I'b) 400 µg | compound (I'b) 600 µg | morphine 60 mg |
|---|---|---|---|---|---|
| Nausea | 17.0 | 29.1 | 49.0 | 64.9 | 66.0 |
| Vomiting | 2.1 | 9.1 | 20.4 | 49.1 | 40.0 |
| Dizziness | 6.4 | 20.0 | 22.4 | 26.3 | 24.0 |
| Somnolence | 2.1 | 1.8 | 10.2 | 14.0 | 16.0 |
| ASAT increased | 2.1 | 1.8 | 6.1 | 1.8 | 2.0 |
| Hot flush | 0 | 1.8 | 4.1 | 7.0 | 4.0 |
| Pruritus | 0 | 0 | 6.1 | 3.5 | 2.0 |
| Hyperhidrosis | 0 | 0 | 0 | 5.3 | 6.0 |

| | | | | | |
|---|---|---|---|---|---|
| 100% = total number of subjects in corresponding treatment group; ASAT: aspartate aminotransferase | | | | | |

It becomes evident from Tables 22 and 23 that all four active treatments were well tolerated under these circumstances and the adverse events that showed up most frequently are in line with what can be expected from µ-opioid receptor agonists. For the patient group that had been treated with compound (I'b), the incidence of adverse events increased with the dose, and at a dose of 600 µg the incidence of adverse events was comparable to that of the morphine patient group.

### EXAMPLE 8:

Clinical studies were conducted to determine the bioavailability of a liquid filled capsule formulation containing compound (I'b) in a dose strength of 400 µg compared to a hemicitrate oral solution of compound (I'b) (400 µg, 400 µg/mL oral solution) in a Macrogol 400 formulation after single oral administration. 24 healthy white male subjects were included in a randomized, open-label, 3-way crossover, single-center clinical trial. The main pharmacokinetic parameters were AUC₀₋ₜ, AUC₀₋₇₂ₕ and Cₘₐₓ.

The results are summarized in Tables 24 to 26.

**Table 24**

| pharmacokinetic parameter | tₘₐₓ* [h] | Cₘₐₓ [pg/mL] | AUC₀₋₇₂ₕ [h·pg/mL] | AUC₀₋ₜ[h·pg/mL] |
|---|---|---|---|---|
| 400 µg/mL oral solution | 6.00 (2.08; 6.00) | 127 ± 52.4 (41.2%) | 2771 ±1376 (49.7%) | 3843 ± 2081 (54.1%) |
| 400 µg capsule | 6.00 (2.08; 10.0) | 131 ± 58.1 (44.2%) | 2814 ±1637 (58.2%) | 3733 ± 2265 (60.7%) |

| | | | | |
|---|---|---|---|---|
| N = 24; The table presents the arithmetic means +/- the standard deviation (coefficient of variation). | | | | |

**Table 25**

| comparison capsules/oral solution | Cₘₐₓ | AUC₀₋₇₂ₕ | AUC₀₋ₜ |
|---|---|---|---|
| 400 µg capsule / 400 µg/mL oral solution | 105% (94.4%-116%) | 105% (96%-116%) | 100% (91.0%-111%) |

**Table 26**

| | total number of subjects (N) | Subjects n | with TEAE(s) % | TEAE(S) e |
|---|---|---|---|---|
| 400 µg capsule | 24 | 14 | 58.3 | 32 |
| 400 µg/mL oral solution | 24 | 18 | 75.0 | 43 |

| | | | | |
|---|---|---|---|---|
| n: number of subjects with at least one TEAE (treatment emergent adverse event); %: corresponding ratio of subjects experiencing TEAE(s); e: number of TEAE(s) | | | | |

Accordingly, the relative bioavailability of the 400 µg capsule and 400 µg/mL oral solution based on AUC₀₋₇₂ₕ was 105%, with 90%-Cl within the 80% to 125% range used for assessing bioequivalence.

Single oral dose administrations of 400 µg of compound (I'b) were safe and well tolerated independent from the galenic formulation. No serious adverse events occured.

### Prophetic examples:

Prophetic examples of pharmaceutical dosage forms according to the invention are provided below. Their compositions are intended to be exemplary and it should be understood that the ingredients, the amount thereof and the procedure to obtain the dosage form may be varied.

According to example 6, hard gelatin capsules containing compound (I'b) in a dosage of, for instance, 40 or 400 µg, can also be produced by means of the following SMEDDS formulations:

| Ingredients [%] | PE-A1 | PE-A2 | PE-A3 | PE-A4 | PE-A5 |
|---|---|---|---|---|---|
| Solutol® HS 15 | 10 | 17.5 | 25 | 32.5 | 40 |
| Miglyol 812 | 60 | 55 | 50 | 40 | 30 |
| Ethanol (abs.) | 30 | 27.5 | 25 | 27.5 | 30 |
| | | | | | |

| | PE-A6 | PE-A7 | PE-A8 | PE-A9 | PE-A10 |
|---|---|---|---|---|---|
| Solutol® HS 15 | 45 | 50 | 65 | 80 | 87.5 |
| Miglyol 812 | 27.5 | 25 | 20 | 15 | 10 |
| Ethanol (abs.) | 27.5 | 25 | 15 | 5 | 2.5 |
| | | | | | |

| Ingredients [%] | PE-B1 | PE-B2 | PE-B3 | PE-B4 | PE-B5 |
|---|---|---|---|---|---|
| Gelucire 44/14 | 50 | 55 | 60 | 62.5 | 65 |
| Labrasol | 25 | 22.5 | 20 | 20 | 20 |
| Capryol 90 | 25 | 22.5 | 20 | 17.5 | 15 |
| | | | | | |

| Ingredients [%] | PE-B6 | PE-B7 | PE-B8 | PE-B9 | PE-B10 |
|---|---|---|---|---|---|
| Gelucire 44/14 | 67.5 | 70 | 75 | 80 | 82.5 |
| Labrasol | 17.5 | 15 | 12.5 | 10 | 7.5 |
| Capryol 90 | 15 | 15 | 12.5 | 10 | 10 |
| | | | | | |

| Ingredients [%] | PE-C1 | PE-C2 | PE-C3 | PE-C4 | PE-C5 |
|---|---|---|---|---|---|
| Cremophor RH 40 | 5 | 12.5 | 20 | 25 | 30 |
| Peceol | 15 | 17.5 | 20 | 20 | 20 |
| Miglyol 812 | 80 | 70 | 60 | 55 | 50 |
| | | | | | |

| Ingredients [%] | PE-C6 | PE-C7 | PE-C8 | PE-C9 | PE-C10 |
|---|---|---|---|---|---|
| Cremophor RH 40 | 35 | 40 | 47.5 | 55 | 60 |
| Peceol | 20 | 20 | 17.5 | 15 | 12.5 |
| Miglyol 812 | 45 | 40 | 35 | 30 | 27.5 |

## Claims

1. A pharmaceutical dosage form for use in the treatment of pain, which contains a pharmacologically active agent according to general formula (I) 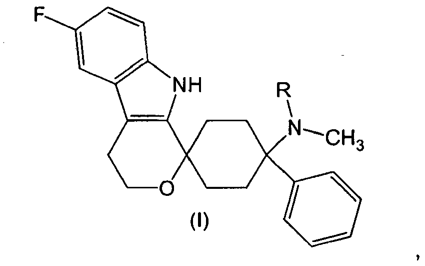 wherein R is -H or -CH₃,
or a physiologically acceptable salt thereof
and a self-emulsifying formulation;
wherein the pharmaceutical dosage form is administered twice daily, once daily or less frequently.

2. The pharmaceutical dosage form for use according to claim 1, wherein the pharmacologically active agent according to general formula (I) is molecularly dispersed.

3. The pharmaceutical dosage form for use according to claim 1 or 2, which comprises a liquid core encapsulated by a solid material, wherein the pharmacologically active agent according to general formula (I) is dispersed in the liquid core.

4. The pharmaceutical dosage form for use according to any of the preceding claims, wherein the self-emulsifying formulation gives (micro)emulsions with an average droplet size smaller than or equal 10 micrometers in presence of aqueous media.

5. The pharmaceutical dosage form for use according to any of the preceding claims, which comprises a surfactant having a HLB value of at least 10.

6. The pharmaceutical dosage form for use according to any of the preceding claims, which comprises a surfactant in an amount of at least 0.001 wt.-%, based on the total weight of the pharmaceutical dosage form.

7. The pharmaceutical dosage form for use according to any of the preceding claims, which comprises a surfactant selected from the group consisting of polyoxyethylene fatty acid esters, partial fatty acid esters of polyoxyethylenesorbitan, and sulfuric acid esters.

8. The pharmaceutical dosage form for use according to any of the preceding claims, wherein the pharmacologically active agent according to general formula (I) has a stereochemistry according to general formula (I') 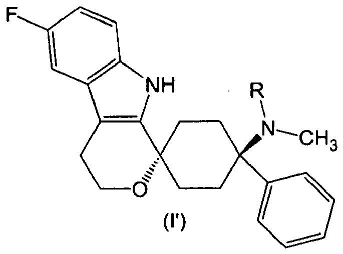 wherein R is defined as in claim 1.

9. The pharmaceutical dosage form for use according to any of the preceding claims, wherein the pharmacologically active agent according to general formula (I) is (1r,4r)-6'-fluoro-N,N-dimethyl-4-phenyl-4',9'-dihydro-3'H-spiro[cyclohexane-1,1'-pyrano[3,4,b]-indol]-4-amine, (1r,4r)-6'-fluoro-N-methyl-4-phenyl-4',9'-dihydro-3'H-spiro[cyclohexane-1,1'-pyrano[3,4,b]indol]-4-amine, or a physiologically acceptable salt thereof.

10. The pharmaceutical dosage form for use according to any of the preceding claims, which releases under *in vitro* conditions in 900 mL artificial gastric juice at pH 1.2 after 30 minutes at least 80 wt.-% of the pharmacologically active agent according to general formula (I), based on the total amount of the pharmacologically active agent according to general formula (I) originally contained in the dosage form.

11. The pharmaceutical dosage form for use according to any of the preceding claims, which contains the pharmacologically active agent according to general formula (I) in a dose of from 10 µg to 50 µg or of from 300 µg to 500 µg.

12. The pharmaceutical dosage form for use according to any of the preceding claims, wherein the pain is selected from acute, visceral, neuropathic or chronic pain.

## Patentansprüche

1. Pharmazeutische Darreichungsform für die Verwendung bei der Behandlung von Schmerzen, welche einen pharmakologisch aktiven Wirkstoff gemäß der allgemeinen Formel (I) enthält 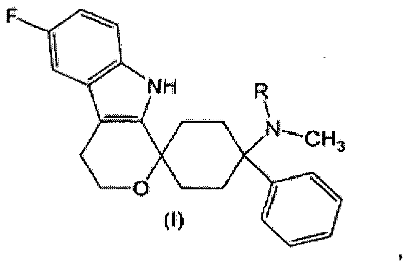 wobei R -H oder -CH₃ ist
oder ein physiologisch annehmbares Salz davon
und eine selbstemulgierende Formulierung;
wobei die pharmazeutische Darreichungsform zweimal täglich, einmal täglich oder weniger häufig verabreicht wird.

2. Pharmazeutische Darreichungsform für die Verwendung nach Anspruch 1, wobei der pharmakologisch aktive Wirkstoff gemäß der allgemeinen Formel (I) molekulardispers verteilt ist.

3. Pharmazeutische Darreichungsform für die Verwendung nach Anspruch 1 oder 2, welche einen flüssigen Kern umfasst, der von einem festen Material eingekapselt wird, wobei der pharmakologisch aktive Wirkstoff gemäß der allgemeinen Formel (I) im flüssigen Kern dispers verteilt ist.

4. Pharmazeutische Darreichungsform für die Verwendung nach einem der vorhergehenden Ansprüche, wobei die selbstemulgierende Formulierung in Anwesenheit wässriger Medien (Mikro-)Emulsionen mit einer mittleren Tröpfchengröße kleiner gleich 10 Mikrometer ergibt.

5. Pharmazeutische Darreichungsform für die Verwendung nach einem der vorhergehenden Ansprüche, welche ein Tensid mit einem HLB-Wert von mindestens 10 umfasst.

6. Pharmazeutische Darreichungsform für die Verwendung nach einem der vorhergehenden Ansprüche, welche ein Tensid in einer Menge von mindestens 0,001 Gew.-% basierend auf dem Gesamtgewicht der pharmazeutischen Darreichungsform umfasst.

7. Pharmazeutische Darreichungsform für die Verwendung nach einem der vorhergehenden Ansprüche, welche ein Tensid umfasst, das aus der aus Polyoxyethylen-Fettsäureestern, Partialfettsäureestern von Polyoxyethylensorbitan und Schwefelsäureestern bestehenden Gruppe ausgewählt ist.

8. Pharmazeutische Darreichungsform für die Verwendung nach einem der vorhergehenden Ansprüche, wobei der pharmakologisch aktive Wirkstoff gemäß der allgemeinen Formel (I) eine Stereochemie gemäß der allgemeinen Formel (I') aufweist 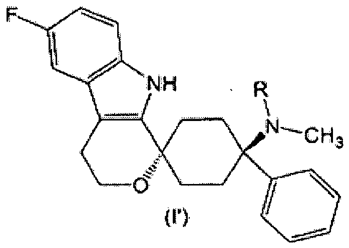 wobei R wie in Anspruch 1 definiert ist.

9. Pharmazeutische Darreichungsform für die Verwendung nach einem der vorhergehenden Ansprüche, wobei der pharmakologisch aktive Wirkstoff gemäß der allgemeinen Formel (I) (1r,4r)-6'-Fluor-N,N-dimethyl-4-phenyl-4',9'-dihydro-3'H-spiro[cyclohexan-1,1'-pyrano[3,4,b]indol]-4-amin, (1r,4r)-6'-Fluor-N-methyl-4-phenyl-4',9'-dihydro-3'H-spiro[cyclohexan-1,1'-pyrano[3,4,b]indol]-4-amin ist oder ein physiologisch annehmbares Salz davon.

10. Pharmazeutische Darreichungsform für die Verwendung nach einem der vorhergehenden Ansprüche, welche unter In-vitro-Bedingungen in 900 ml künstlichem Magensaft bei pH-Wert 1,2 nach 30 Minuten mindestens 80 Gew.-% des pharmakologisch aktiven Wirkstoffs gemäß der allgemeinen Formel (I) basierend auf der ursprünglich in der Darreichungsform enthaltenen Gesamtmenge des pharmakologisch aktiven Wirkstoffs gemäß der allgemeinen Formel (I) freisetzt.

11. Pharmazeutische Darreichungsform für die Verwendung nach einem der vorhergehenden Ansprüche, welche den pharmakologisch aktiven Wirkstoff gemäß der allgemeinen Formel (I) in einer Dosis von 10 µg bis 50 µg oder von 300 µg bis 500 µg enthält.

12. Pharmazeutische Darreichungsform für die Verwendung nach einem der vorhergehenden Ansprüche, wobei der Schmerz aus akuten, viszeralen, neuropathischen oder chronischen Schmerzen ausgewählt ist.

## Revendications

1. Forme posologique pharmaceutique pour une utilisation dans le traitement de la douleur, qui contient un agent pharmacologiquement actif répondant à la formule générale (I) 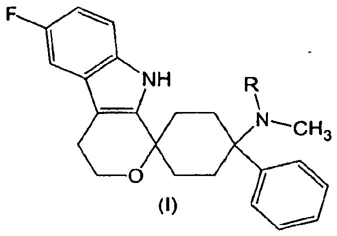 dans laquelle R représente -H ou un groupe -CH₃,
ou un de ses sels physiologiquement acceptables et une formulation auto-émulsionnable ;
ladite forme posologique pharmaceutique étant administrée deux fois par jour, une fois par jour ou moins fréquemment.

2. Forme posologique pharmaceutique pour une utilisation suivant la revendication 1, dans laquelle l'agent pharmacologiquement actif répondant à la formule générale (I) est en dispersion moléculaire.

3. Forme posologique pharmaceutique pour une utilisation suivant la revendication 1 ou 2, qui comprend un noyau liquide encapsulé par une matière solide, dans laquelle l'agent pharmacologiquement actif répondant à la formule générale (I) est dispersé dans le noyau liquide.

4. Forme posologique pharmaceutique pour une utilisation suivant l'une quelconque des revendications précédentes, dans laquelle la formulation auto-émulsionnable donne des (micro)émulsions ayant un diamètre moyen de gouttelettes inférieur ou égal à 10 micromètres en présence de milieux aqueux.

5. Forme posologique pharmaceutique pour une utilisation suivant l'une quelconque des revendications précédentes, qui comprend un agent tensioactif ayant une valeur d'équilibre hydrophile-lipophile (HLB) d'au moins 10.

6. Forme posologique pharmaceutique pour une utilisation suivant l'une quelconque des revendications précédentes, qui comprend un agent tensioactif en une quantité d'au moins 0,001 % en poids, sur la base du poids total de la forme posologique pharmaceutique.

7. Forme posologique pharmaceutique pour une utilisation suivant l'une quelconque des revendications précédentes, qui comprend un agent tensioactif choisi dans le groupe consistant en des esters d'acides gras de polyoxyéthylène, des esters partiels d'acides gras de polyoxyéthylène-sorbitanne, et des esters d'acide sulfurique.

8. Forme posologique pharmaceutique pour une utilisation suivant l'une quelconque des revendications précédentes, dans laquelle l'agent pharmacologiquement actif répondant à la formule générale (I) a une stéréochimie correspondant à la formule générale (I') 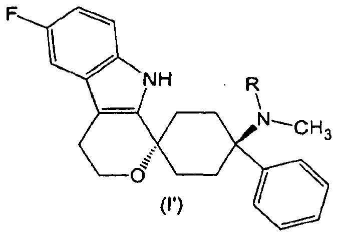 dans laquelle R est tel que défini dans la revendication 1.

9. Forme posologique pharmaceutique pour une utilisation suivant l'une quelconque des revendications précédentes, dans laquelle l'agent pharmacologiquement actif répondant à la formule générale (I) est la (1r,4r)-6'-fluoro-N,N-diméthyl-4-phényl-4',9'-dihydro-3'H-spiro-[cyclohexane-1,1'-pyranno[3,4,b]-indole]-4-amine, la (1r,4r)-6'-fluoro-N-méthyl-4-phényl-4',9'-dihydro-3'H-spiro[cyclo-hexane-1,1'-pyranno[3,4,b]-indole]-4-amine ou un de leurs sels physiologiquement acceptables.

10. Forme posologique pharmaceutique pour une utilisation suivant l'une quelconque des revendications précédentes, qui libère dans des conditions *in vitro* dans 900 ml de suc gastrique artificiel à pH 1,2 au bout de 30 minutes au moins 80 % en poids de l'agent pharmacologiquement actif répondant à la formule générale (I), sur la base de la quantité totale de l'agent pharmacologiquement actif répondant à la formule générale (I) présent initialement dans la forme posologique.

11. Forme posologique pharmaceutique pour une utilisation suivant l'une quelconque des revendications précédentes, qui contient l'agent pharmacologiquement actif répondant à la formule générale (I) dans une dose de 10 µg à 50 µg ou de 300 µg à 500 µg.

12. Forme posologique pharmaceutique pour une utilisation suivant l'une quelconque des revendications précédentes, la douleur étant choisie entre la douleur aiguë, la douleur viscérale, la douleur neuropathique et la douleur chronique.
